# EUROPÄISCHE PATENTANMELDUNG

(11) **EP 1 844 762 A2**
(43) Veröffentlichungstag der Anmeldung: **17.10.2007**
(21) Anmeldenummer: 07005550.4
(22) Anmeldetag: 19.03.2007
(51) Int. Cl.: A61K 8/891, A61K 8/894, A61Q 5/02, A61Q 5/12

(54) **Kosmetisches Mittel mit mindestens 2 Silikonen**

(30) Priorität: 11.04.2006 DE 102006016908
(71) Anmelder: Henkel Kommanditgesellschaft auf Aktien, 40589 Düsseldorf (DE)
(72) Erfinder: Goddinger, Dieter, 25336 Klein Nordende (DE); Schröder, Thomas, 22395 Hamburg (DE)

(57) **Zusammenfassung**

Kosmetische Mittel, die - bezogen auf ihr Gewicht - 0,01 bis 10 Gew.-% mindestens eines Silikons der Formel Si-I

(CH₃)₃Si-[O-Si(CH₃)₂]ₓ-O-Si(CH₃)₃ (Si-I),

in der x für eine Zahl von 0 bis 5000, vorzugsweise von 10 bis 2500, weiter bevorzugt von 50 bis 1500 und insbesondere 100 bis 1000, steht, und 0,01 bis 10 Gew.-% mindestens eines Silikons der Formel Si-II

(CH₃)₃Si-[O-Si(CH₃)₂]ₓ-[0-(H₃C)Si((CH₂)ₖ(CH₂CH₂O)ₘ(CH₂CH₂CH₂O)ₘ(CH₂CH₂CH₂Oₙ)]_{y}-O-Si(CH₃)₃ (Si-II),

in der
- k für Werte von 1 bis 20, vorzugsweise von 2 bis 10 und insbesondere für 2, 3, 4, 5, 6 steht;
- m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
- n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
- x bzw. y jeweils unabhängig voneinander für eine Zahl von 0 bis 5000, vorzugsweise von 10 bis 2500, weiter bevorzugt von 50 bis 1500 und insbesondere 100 bis 1000 stehen,
mit der Maßgabe, daß (m + n) nicht Null ist
enthalten, führen zu einer verbesserten Abscheidung der Silikone auf den behandelten Körperoberflächen, insbesondere den Haaren.

## Beschreibung

Die vorliegende Erfindung betrifft kosmetische Zusammensetzungen, d.h. kosmetische oder dermatologische Zusammensetzungen zur Anwendung auf der Haut, insbesondere menschlicher Haut, sowie zur Anwendung auf Keratinfasern, insbesondere menschlichen Haaren, und deren Verwendung.

Die erfindungsgemäßen Zusammensetzungen sind kosmetische Mittel. Während man früher zwischen Mitteln zur Pflege des menschlichen Körpers und solchen zur Verschönerung seines Aussehens unterschied nach "Körperpflegemitteln" und "dekorativen Kosmetika", werden diese Produkte heute zusammengefaßt definiert als *"kosmetische Mittel"* oder kosmetische Zusammensetzungen.
Angesichts der Allgemeinzugänglichkeit der kosmetischen Mittel und ihrer Anwendung am menschlichen Körper besteht zum Schutz des Verbrauchers in Deutschland und der Europäischen Union ein umfangreiches Regelwerk. Gesetzliche Grundlage in Deutschland ist das Lebensmittel- und Bedarfsgegenstände-Gesetz (LMBG), das kosmetische Mittel in § 4 wie folgt definiert:
"(1) kosmetische Mittel im Sinne dieses Gesetzes sind Stoffe oder Zubereitungen aus Stoffen, die dazu bestimmt sind, äußerlich am Menschen oder in seiner Mundhöhle zur Reinigung, Pflege oder zur Beeinflussung des Aussehens oder des Körpergeruchs oder zur Vermittlung von Geruchseindrücken angewendet zu werden, es sei denn, daß sie überwiegend dazu bestimmt sind, Krankheiten, Leiden, Körperschäden oder krankhafte Beschwerden zu lindern oder zu beseitigen." [§ 4 LMBG ist bisher nicht an die EU-Kosmetikrichtlinie angepasst, die Kosmetika sechs Funktionen (reinigen, parfümieren, Aussehen verändern, Körpergeruch beeinflussen, schützen und in gutem Zustand halten) zuspricht.]
"(2) Den kosmetischen Mitteln stehen Stoffe oder Zubereitungen aus Stoffen zur Reinigung oder Pflege von Zahnersatz gleich.
(3) Als kosmetische Mittel gelten nicht Stoffe oder Zubereitungen aus Stoffen, die zur Beeinflussung der Körperformen bestimmt sind."

Je nach Anwendungsgebiet unterscheidet man daher ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- u. Mundpflege (Zahn- und Mundpflegemittel, Gebißpflegemittel, Prothesenhaftmittel) und solche zur Haarpflege (Haarwaschmittel, Haarpflegemittel, Haarverfestigungsmittel, Haarverformungsmittel, Mittel zur Farbänderung).

Kosmetische Zusammensetzungen im Sinne der vorliegenden Erfindung sind daher beispielsweise ausgewählt aus der Gruppe der Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Bei allen diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Es besteht daher auf dem Gebiet der Kosmetik ständig der Wunsch, neue Einsatzstoffe bereitzustellen, die in einzelnen, vorzugsweise in allen Produktkategorien vorteilhafte Wirkungen entfalten.

Es wurde nun gefunden, daß sich bestimmte Silikone besser in kosmetische Mittel einarbeiten lassen und besser auf den behandelten Körperoberflächen, insbesondere den Haaren, abgeschieden werden, wenn man eine Kombination von mindestens zwei verschiedenen Silikonen in die Mittel einarbeitet. Besonders ausgeprägt sind diese Effekte auf dem Haar und der Kopfhaut, so daß bevorzugte erfindungsgemäße kosmetische Mittel Haarbehandlungsmittel sind.

Gegenstand der vorliegenden Erfindung sind in einer ersten Ausführungsform kosmetische Mittel, die - bezogen auf ihr Gewicht -
a) 0,01 bis 10 Gew.-% mindestens eines Silikons der Formel Si-I

   **(CH₃)₃Si-[O-Si(CH₃)₂]ₓ-O-Si(CH₃)₃** **(Si-I),**

   in der x für eine Zahl von 0 bis 5000, vorzugsweise von 10 bis 2500, weiter bevorzugt von 50 bis 1500 und insbesondere 100 bis 1000, steht, und
b) 0,01 bis 10 Gew.-% mindestens eines Silikons der Formel Si-II

   **(CH₃)₃Si-[O-Si(CH₃)₂]ₓ-[O-(H₃C)Si((CH₂)ₖCH₂CH₂O)ₘ(CH₂CH₂CH₂O)ₙ)]_{y}-O-Si(CH₃)₃** **(Si-II),**

   in der
   - k für Werte von 1 bis 20, vorzugsweise von 2 bis 10 und insbesondere für 2, 3, 4, 5, 6 steht;
   - m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
   - n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
   - x bzw. y jeweils unabhängig voneinander für eine Zahl von 0 bis 5000, vorzugsweise von 10 bis 2500, weiter bevorzugt von 50 bis 1500 und insbesondere 100 bis 1000 stehen,
   mit der Maßgabe, daß (m + n) nicht Null ist
enthalten.

Als ersten wesentlichen Bestandteil enthalten die erfindungsgemäßen Mittel mindestens ein Silikon der Formel Si-I

**(CH₃)₃Si-[O-Si(CH₃)₂]ₓ-O-Si(CH₃)₃** **(Si-I),**

in der x für eine Zahl von 0 bis 5000, vorzugsweise von 10 bis 2500, weiter bevorzugt von 50 bis 1500 und insbesondere 100 bis 1000, steht.

Diese einfachen, linear-polymeren Silikone sind nach dem Schema (R₂SiO)ₓ aufgebaut. Systematisch werden solche Silikone als *Polyorganosiloxane* bezeichnet; diese Namensbildung basiert auf der Benennung der Si-O-Si-Bindung als Siloxan-Bindung und hat sich in der wissenschaftlichen Literatur eingebürgert. Ein Polymer der allg. Formel Si-I wird als Poly(dimethylsiloxan) bezeichnet, kann aber nach den IUPAC-Regeln zur Benennung linearer organischer Polymerer auch Poly[oxy(dimethylsilylen)], nach den Regeln für anorganische Makromoleküle *caten*a-Poly[(dimethylsilicium)-µ-oxo] genannt werden; der internat. Freiname der Verbindungen Ist Dimethicon, nach INCI werden diese Silicone als Dimethicone bezeichnet.

Erfindungsgemäß bevorzugte Mittel enthalten Dimethicone, d.h. Silikone der Formel Si-I, in engeren Mengen. Hier sind erfindungsgemäße kosmetische Mittel bevorzugt, die - bezogen auf ihr Gewicht - 0,02 bis 8,5 Gew.-%, vorzugsweise 0,1 bis 7,5 Gew.-%, besonders bevorzugt 0,25 bis 5 Gew.-%, weiter bevorzugt 0,25 bis 4 Gew.-% und insbesondere 0,3 bis 2,5 Gew.-% mindestens eines Silikons der Formel Si-I enthalten.

Die Silikone der Formel Si-I können Oligomeriserungs- bzw. Polymerisationsgrade zwischen 0 (entsprechend (CH₃)₃Si-O-Si(CH₃)₃) und 500 aufweisen, wobei bevorzugte Oligomeriserungs- bzw. Polymerisationsgrade im Bereich von 10 bis 2500, weiter bevorzugt von 50 bis 1500 und insbesondere von 100 bis 1000 liegen. Naturgemäß variiert das Molekulargewicht der Dimethicone mit dem Oligomeriserungs- bzw. Polymerisationsgrad und liegt zwischen 162 Dalton (für den oben genannten Oligomerisierungsgrad 0) und einigen 100 kDa für hohe Polymerisationsgrade, beispielsweise bei ca. 60 kDa für einen Polymerisationsgrad von 810.

Die Viskosität der erfindungsgemäß eingesetzten Dimethicone ist nahezu temperaturunabhängig und variiert mit der Molmasse, wobei hochmolekulare Dimethicone höhere Viskositäten aufweisen als niedermolekulare. Bei üblichen Meßbedingungen (20°C, 1013,25 mbar) weist (CH₃)₃Si-O-Si(CH₃)₃ eine Viskosität von 0,65 mm²/s (Centistokes, cSt) auf, wobei diese Viskosität auch kaum Abhängigkeit von der Meßapparatur zeigt. Beispielsweise kann die Viskosität bei den oben genannten normalen Meßbedingungen mit einem Brookfield-Viskosimeter LVT, Spindel 2, 30 U/min gemessen werden. Viskositätsangaben im Rahmen der vorliegenden Anmeldung beziehen sich immer auf 20°C und 1013,25 mbar sowie auf Apparaturen, bei denen bei diesen Bedingungen eine Viskosität des (CH₃)₃Si-O-Si(CH₃)₃ von 0,65 mm²/s (Centistokes, cSt) gemessen wird.

Erfindungsgemäß besonders bevorzugte kosmetische Mittel sind dadurch gekennzeichnet, daß sie als Silikon der Formel Si-I ein Dimethicone enthalten, das eine Viskosität von 10 bis 1.000.000 cSt, vorzugsweise von 100 bis 600.000 cSt, besonders bevorzugt von 1000 bis 300.000 cSt, weiter bevorzugt von 5000 bis 200.000 cSt und insbesondere von 10.000 bis 70.000 cSt (gemessen bei 20°C) aufweist.

Besonders bevorzugte erfindungsgemäß einsetzbare Dimethicone weisen Polymerisationsgrade zwischen 700 und 1400 auf. Insbesondere bevorzugte erfindungsgemäße kosmetische Mittel sind dadurch gekennzeichnet, daß mindestens 25 Gew.-%, vorzugsweise mindestens 50 Gew.-%, besonders bevorzugt mindestens 75 Gew.-%, weiter bevorzugt mindestens 95 Gew.-% und insbesondere 100 Gew.-% der im Mittel enthaltenen Silikone der Formel Si-I ausgewählt ist/sind aus Silikonen der Formel Si-I

(CH₃)₃Si-[O-Si(CH₃)₂]ₓ-O-Si(CH₃)₃ (Si-I)

in der x für eine Zahl aus der Gruppe 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, 900, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1018, 1019, 1020, 1021, 1022, 1023, 1024, 1025, 1026, 1027, 1028, 1029, 1030, 1031, 1032, 1033, 1034, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1058, 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1083, 1084, 1085, 1086, 1087, 1088, 1089, 1090, 1091, 1092, 1093, 1094, 1095, 1096, 1097, 1098, 1099, 1100 steht.

Erfindungsgemäß besonders bevorzugte Mittel enthalten demnach Silikone der Formel Si-I, die ausgerwählt sind aus den Dimethiconen (CH₃)₃Si-[O-Si(CH₃)₂]₈₀₀-O-Si(CH₃)₃, (CH₃)₃Si-[O-Si(CH₃)₂]₈₀₁-O-Si(CH₃)₃, (CH₃)₃Si-[O-Si(CH₃)₂]₈₀₂-O-Si(CH₃)₃, (CH₃)₃Si-[O-Si(CH₃)₂]₈₀₃-O-Si(CH₃)₃, (CH₃)₃Si-[O-Si(CH₃)₂]₈₀₄-O-Si(CH₃)₃, (CH₃)₃Si-[O-Si(CH₃)₂]₈₀₅-O-Si(CH₃)₃, (CH₃)₃Si-[O-Si(CH₃)₂]₈₀₆-O-Si(CH₃)₃, (CH₃)₃Si-[O-Si(CH₃)₂]₈₀₇-O-Si(CH₃)₃, (CH₃)₃Si-[O-Si(CH₃)₂]₈₀₈-O-Si(CH₃)₃, (CH₃)₃Si-[O-Si(CH₃)₂]₈₀₉-O-Si(CH₃)₃, (CH₃)₃Si-[O-Si(CH₃)₂]₈₁₀-O-Si(CH₃)₃, (CH₃)₃Si-[O-Si(CH₃)₂]₈₁₁-O-Si(CH₃)₃, (CH₃)₃Si-[O-Si(CH₃)₂]₈₁₂-O-Si(CH₃)₃, (CH₃)₃Si-[O-Si(CH₃)₂]₈₁₃-O-Si(CH₃)₃, (CH₃)₃Si-[O-Si(CH₃)₂]₈₁₄-O-Si(CH₃)₃, (CH₃)₃Si-[O-Si(CH₃)₂]₈₁₅-O-Si(CH₃)₃, (CH₃)₃Si-[O-Si(CH₃)₂]₈₁₆-O-Si(CH₃)₃, (CH₃)₃Si-[O-Si(CH₃)₂]₈₁₇-O-Si(CH₃)₃, (CH₃)₃Si-[O-Si(CH₃)₂]₈₁₈-O-Si(CH₃)₃, (CH₃)₃Si-[O-Si(CH₃)₂]₈₁₉-O-Si(CH₃)₃, (CH₃)₃Si-[O-Si(CH₃)₂]₈₂₀-O-Si(CH₃)₃, (CH₃)₃Si-[O-Si(CH₃)₂]₈₂₁-O-Si(CH₃)₃, (CH₃)₃Si-[O-Si(CH₃)₂]₈₂₂-O-Si(CH₃)₃, (CH₃)₃Si-[O-Si(CH₃)₂]₈₂₃-O-Si(CH₃)₃, (CH₃)₃Si-[O-Si(CH₃)₂]₈₂₄-O-Si(CH₃)₃, (CH₃)₃Si-[O-Si(CH₃)₂]₈₂₅-O-Si(CH₃)₃, (CH₃)₃Si-[O-Si(CH₃)₂]₈₂₆-O-Si(CH₃)₃, (CH₃)₃Si-[O-Si(CH₃)₂]₈₂₇-O-Si(CH₃)₃, (CH₃)₃Si-[O-Si(CH₃)₂]₈₂₈-O-Si(CH₃)₃, (CH₃)₃Si-[O-Si(CH₃)₂]₈₂₉-O-Si(CH₃)₃, (CH₃)₃Si-[O-Si(CH₃)₂]₈₃₀-O-Si(CH₃)₃, (CH₃)₃Si-[O-Si(CH₃)₂]₈₃₁-O-Si(CH₃)₃, (CH₃)₃Si-[O-Si(CH₃)₂]₈₃₂-O-Si(CH₃)₃, (CH₃)₃Si-[O-Si(CH₃)₂]₈₃₃-O-Si(CH₃)₃, (CH₃)₃Si-[O-Si(CH₃)₂]₈₃₄-O-Si(CH₃)₃, (CH₃)₃Si-[O-Si(CH₃)₂]₈₃₅-O-Si(CH₃)₃. (CH₃)₃Si-[O-Si(CH₃)₂]₈₃₆-O-Si(CH₃)₃, (CH₃)₃Si-[O-Si(CH₃)₂]₈₃₇-O-Si(CH₃)₃, (CH₃)₃Si-[O-Si(CH₃)₂]₈₃₈-O-Si(CH₃)₃, (CH₃)₃Si-[O-Si(CH₃)₂]₈₃₉-O-Si(CH₃)₃, (CH₃)₃Si-[O-Si(CH₃)₂]₈₄₀-O-Si(CH₃)₃, (CH₃)₃Si-[O-Si(CH₃)₂]₈₄₁-O-Si(CH₃)₃, (CH₃)₃Si-[O-Si(CH₃)₂]₈₄₂-O-Si(CH₃)₃, (CH₃)₃Si-[O-Si(CH₃)₂]₈₄₃-O-Si(CH₃)₃, (CH₃)₃Si-[O-Si(CH₃)₂]₈₄₄-O-Si(CH₃)₃, (CH₃)₃Si-[O-Si(CH₃)₂]₈₄₅-O-Si(CH₃)₃, (CH₃)₃Si-[O-Si(CH₃)₂]₈₄₆-O-Si(CH₃)₃, (CH₃)₃Si-[O-Si(CH₃)₂]₈₄₇-O-Si(CH₃)₃, (CH₃)₃Si-[O-Si(CH₃)₂]₈₄₈-O-Si(CH₃)₃, (CH₃)₃Si-[O-Si(CH₃)₂]₈₄₉-O-Si(CH₃)₃, (CH₃)₃Si-[O-Si(CH₃)₂]₈₅₀-O-Si(CH₃)₃, (CH₃)₃S)-[O-Si(CH₃)₂]₈₅₁-O-Si(CH₃)₃, (CH₃)₃Si-[O-Si(CH₃)₂]₈₅₂-O-Si(CH₃)₃, (CH₃)₃Si-[O-Si(CH₃)₂]₈₅₃-O-Si(CH₃)₃, (CH₃)₃Si-[O-Si(CH₃)₂]₈₅₄-O-Si(CH₃)₃, (CH₃)₃Si-[O-Si(CH₃)₂]₈₅₅-O-Si(CH₃)₃, (CH₃)₃Si-[O-Si(CH₃)₂]₈₅₆-O-Si(CH₃)₃, (CH₃)₃Si-[O-Si(CH₃)₂]₈₅₇-O-Si(CH₃)₃, (CH₃)₃Si-[O-Si(CH₃)₂]₈₅₈-O-Si(CH₃)₃, (CH₃)₃Si-[O-Si(CH₃)₂]₈₅₉-O-Si(CH₃)₃, (CH₃)₃Si-[O-Si(CH₃)₂]₈₆₀-O-Si(CH₃)₃, (CH₃)₃Si-[O-Si(CH₃)₂]₈₆₁-O-Si(CH₃)₃, (CH₃)₃Si-[O-Si(CH₃)₂]₈₆₂-O-Si(CH₃)₃, (CH₃)₃Si-[O-Si(CH₃)₂]₈₆₃-O-Si(CH₃)₃, (CH₃)₃Si-[O-Si(CH₃)₂]₈₆₄-O-Si(CH₃)₃, (CH₃)₃Si-[O-Si(CH₃)₂]₈₆₅-O-Si(CH₃)₃, (CH₃)₃Si-[O-Si(CH₃)₂]₈₆₆-O-Si(CH₃)₃, (CH₃)₃Si-[O-Si(CH₃)₂]₈₆₇-O-Si(CH₃)₃, (CH₃)₃Si-[O-Si(CH₃)₂]₈₆₈-O-Si(CH₃)₃, (CH₃)₃Si-[O-Si(CH₃)₂]₈₆₉-O-Si(CH₃)₃, (CH₃)₃Si-[O-Si(CH₃)₂]₈₇₀-O-Si(CH₃)₃, (CH₃)₃Si-[O-Si(CH₃)₂]₈₇₁-O-Si(CH₃)₃, (CH₃)₃Si-[O-Si(CH₃)₂]₈₇₂-O-Si(CH₃)₃, (CH₃)₃Si-[O-Si(CH₃)₂]₈₇₃-O-Si(CH₃)₃, (CH₃)₃Si-[O-Si(CH₃)₂]₈₇₄-O-Si(CH₃)₃, (CH₃)₃Si-[O-Si(CH₃)₂]₈₇₅-O-Si(CH₃)₃. (CH₃)₃Si-[O-Si(CH₃)₂]₈₇₆-O-Si(CH₃)₃, (CH₃)₃Si-[O-Si(CH₃)₂]₈₇₇-O-Si(CH₃)₃, (CH₃)₃Si-[O-Si(CH₃)₂]₈₇₈-O-Si(CH₃)₃, (CH₃)₃Si-[O-Si(CH₃)₂]₈₇₉-O-Si(CH₃)₃, (CH₃)₃Si-[O-Si(CH₃)₂]₈₈₀-O-Si(CH₃)₃, (CH₃)₃Si-[O-Si(CH₃)₂]₈₈₁-O-Si(CH₃)₃, (CH₃)₃Si-[O-Si(CH₃)₂]₈₈₂-O-Si(CH₃)₃, (CH₃)₃Si-[O-Si(CH₃)₂]₈₈₃-O-Si(CH₃)₃, (CH₃)₃Si-[O-Si(CH₃)₂]₈₈₄-O-Si(CH₃)₃, (CH₃)₃Si-[O-Si(CH₃)₂]₈₈₅-O-Si(CH₃)₃, (CH₃)₃Si-[O-Si(CH₃)₂]₈₈₆-O-Si(CH₃)₃, (CH₃)₃Si-[O-Si(CH₃)₂]₈₈₇-O-Si(CH₃)₃, (CH₃)₃Si-[O-Si(CH₃)₂]₈₈₈-O-Si(CH₃)₃, (CH₃)₃Si-[O-Si(CH₃)₂]₈₈₉-O-Si(CH₃)₃, (CH₃)₃Si-[O-Si(CH₃)₂]₈₉₀-O-Si(CH₃)₃, (CH₃)₃Si-[O-Si(CH₃)₂]₈₉₁-O-Si(CH₃)₃, (CH₃)₃Si-[O-Si(CH₃)₂]₈₉₂-O-Si(CH₃)₃, (CH₃)₃S)-[O-Si(CH₃)₂]₈₉₃-O-Si(CH₃)₃, (CH₃)₃Si-[O-Si(CH₃)₂]₈₉₄-O-Si(CH₃)₃, (CH₃)₃Si-[O-Si(CH₃)₂]₈₉₅-O-Si(CH₃)₃, (CH₃)₃Si-[O-Si(CH₃)₂]₈₉₆-O-Si(CH₃)₃, (CH₃)₃Si-[O-Si(CH₃)₂]₈₉₇-O-Si(CH₃)₃, (CH₃)₃Si-[O-Si(CH₃)₂]₈₉₈-O-Si(CH₃)₃, (CH₃)₃Si-[O-Si(CH₃)₂]₈₉₉-O-Si(CH₃)₃, (CH₃)₃Si-[O-Si(CH₃)₂]₉₀₀-O-Si(CH₃)₃, (CH₃)₃Si-[O-Si(CH₃)₂]₉₀₁-O-Si(CH₃)₃, (CH₃)₃Si-[O-Si(CH₃)₂]₉₀₂-O-Si(CH₃)₃, (CH₃)₃Si-[O-Si(CH₃)₂]₉₀₃-O-Si(CH₃)₃, (CH₃)₃Si-[O-Si(CH₃)₂]₉₀₄-O-Si(CH₃)₃, (CH₃)₃Si-[O-Si(CH₃)₂]₉₀₅-O-Si(CH₃)₃, (CH₃)₃Si-[O-Si(CH₃)₂]₉₀₆-O-Si(CH₃)₃, (CH₃)₃Si-[O-Si(CH₃)₂]₉₀₇-O-Si(CH₃)₃, (CH₃)₃Si-[O-Si(CH₃)₂]₉₀₈-O-Si(CH₃)₃, (CH₃)₃Si-[O-Si(CH₃)₂]₉₀₉-O-Si(CH₃)₃, (CH₃)₃Si-[O-Si(CH₃)₂]₉₁₀-O-Si(CH₃)₃, (CH₃)₃Si-[O-Si(CH₃)₂]₉₁₁-O-Si(CH₃)₃, (CH₃)₃Si-[O-Si(CH₃)₂]₉₁₂-O-Si(CH₃)₃, (CH₃)₃Si-[O-Si(CH₃)₂]₉₁₃-O-Si(CH₃)₃, (CH₃)₃Si-[O-Si(CH₃)₂]₉₁₄-O-Si(CH₃)₃, (CH₃)₃Si-[O-Si(CH₃)₂]₉₁₅-O-Si(CH₃)₃, (CH₃)₃Si-[O-Si(CH₃)₂]₉₁₆-O-Si(CH₃)₃, (CH₃)₃Si-[O-Si(CH₃)₂]₉₁₇-O-Si(CH₃)₃, (CH₃)₃Si-[O-Si(CH₃)₂]₉₁₈-O-Si(CH₃)₃, (CH₃)₃Si-[O-Si(CH₃)₂]₉₁₉-O-Si(CH₃)₃, (CH₃)₃Si-[O-Si(CH₃)₂]₉₂₀-O-Si(CH₃)₃, (CH₃)₃Si-[O-Si(CH₃)₂]₉₂₁-O-Si(CH₃)₃, (CH₃)₃Si-[O-Si(CH₃)₂]₉₂₂-O-Si(CH₃)₃, (CH₃)₃Si-[O-Si(CH₃)₂]₉₂₃-O-Si(CH₃)₃, (CH₃)₃Si-[O-Si(CH₃)₂]₉₂₄-O-Si(CH₃)₃, (CH₃)₃Si-[O-Si(CH₃)₂]₉₂₅-O-Si(CH₃)₃, (CH₃)₃Si-[O-Si(CH₃)₂]₉₂₆-O-Si(CH₃)₃, (CH₃)₃Si-[O-Si(CH₃)₂]₉₂₇-O-Si(CH₃)₃, (CH₃)₃Si-[O-Si(CH₃)₂]₉₂₈-O-Si(CH₃)₃, (CH₃)₃Si-[O-Si(CH₃)₂]₉₂₉-O-Si(CH₃)₃, (CH₃)₃Si-[O-Si(CH₃)₂]₉₃₀-O-Si(CH₃)₃, (CH₃)₃Si-[O-Si(CH₃)₂]₉₃₁-O-Si(CH₃)₃, (CH₃)₃Si-[O-Si(CH₃)₂]₉₃₂-O-Si(CH₃)₃, (CH₃)₃Si-[O-Si(CH₃)₂]₉₃₃-O-Si(CH₃)₃, (CH₃)₃Si-[O-Si(CH₃)₂]₉₃₄-O-Si(CH₃)₃, (CH₃)₃Si-[O-Si(CH₃)₂]₉₃₅-O-Si(CH₃)₃, (CH₃)₃Si-[O-Si(CH₃)₂]₉₃₆-O-Si(CH₃)₃, (CH₃)₃Si-[O-Si(CH₃)₂]₉₃₇-O-Si(CH₃)₃, (CH₃)₃Si-[O-Si(CH₃)₂]₉₃₈-O-Si(CH₃)₃, (CH₃)₃Si-[O-Si(CH₃)₂]₉₃₉-O-Si(CH₃)₃, (CH₃)₃Si-[O-Si(CH₃)₂]₉₄₀-O-Si(CH₃)₃, (CH₃)₃Si-[O-Si(CH₃)₂]₉₄₁-O-Si(CH₃)₃, (CH₃)₃Si-[O-Si(CH₃)₂]₉₄₂-O-Si(CH₃)₃, (CH₃)₃Si-[O-Si(CH₃)₂]₉₄₃-O-Si(CH₃)₃, (CH₃)₃Si-[O-Si(CH₃)₂]₉₄₄-O-Si(CH₃)₃, (CH₃)₃Si-[O-Si(CH₃)₂]₉₄₅-O-Si(CH₃)₃, (CH₃)₃Si-[O-Si(CH₃)₂]₉₄₆-O-Si(CH₃)₃, (CH₃)₃Si-[O-Si(CH₃)₂]₉₄₇-O-Si(CH₃)₃, (CH₃)₃Si-[O-Si(CH₃)₂]₉₄₈-O-Si(CH₃)₃, (CH₃)₃Si-[O-Si(CH₃)₂]₉₄₉-O-Si(CH₃)₃, (CH₃)₃Si-[O-Si(CH₃)₂]₉₅₀-O-Si(CH₃)₃, (CH₃)₃Si-[O-Si(CH₃)₂]₉₅₁-O-Si(CH₃)₃, (CH₃)₃Si-[O-Si(CH₃)₂]₉₅₂-O-Si(CH₃)₃, (CH₃)₃Si-[O-Si(CH₃)₂]₉₅₃-O-Si(CH₃)₃, (CH₃)₃Si-[O-Si(CH₃)₂]₉₅₄-O-Si(CH₃)₃, (CH₃)₃Si-[O-Si(CH₃)₂]₉₅₅-O-Si(CH₃)₃, (CH₃)₃Si-[O-Si(CH₃)₂]₉₅₆-O-Si(CH₃)₃, (CH₃)₃Si-[O-Si(CH₃)₂]₉₅₇-O-Si(CH₃)₃, (CH₃)₃Si-[O-Si(CH₃)₂]₉₅₈-O-Si(CH₃)₃, (CH₃)₃Si-[O-Si(CH₃)₂]₉₅₉-O-Si(CH₃)₃, (CH₃)₃Si-[O-Si(CH₃)₂]₉₆₀-O-Si(CH₃)₃, (CH₃)₃Si-[O-Si(CH₃)₂]₉₆₁-O-Si(CH₃)₃, (CH₃)₃Si-[O-Si(CH₃)₂]₉₆₂-O-Si(CH₃)₃, (CH₃)₃Si-[O-Si(CH₃)₂]₉₆₃-O-Si(CH₃)₃, (CH₃)₃Si-[O-Si(CH₃)₂]₉₆₄-O-Si(CH₃)₃, (CH₃)₃Si-[O-Si(CH₃)₂]₉₆₅-O-Si(CH₃)₃, (CH₃)₃Si-[O-Si(CH₃)₂]₉₆₆-O-Si(CH₃)₃, (CH₃)₃Si-[O-Si(CH₃)₂]₉₆₇-O-Si(CH₃)₃, (CH₃)₃Si-[O-Si(CH₃)₂]₉₆₈-O-Si(CH₃)₃, (CH₃)₃Si-[O-Si(CH₃)₂]₉₆₉-O-Si(CH₃)₃, (CH₃)₃Si-[O-Si(CH₃)₂]₉₇₀-O-Si(CH₃)₃, (CH₃)₃Si-[O-Si(CH₃)₂]₉₇₁-O-Si(CH₃)₃, (CH₃)₃S)-[O-Si(CH₃)₂]₉₇₂-O-Si(CH₃)₃, (CH₃)₃Si-[O-Si(CH₃)₂]₉₇₃-O-Si(CH₃)₃, (CH₃)₃Si-[O-Si(CH₃)₂]₉₇₄-O-Si(CH₃)₃, (CH₃)₃Si-[O-Si(CH₃)₂]₉₇₅-O-Si(CH₃)₃, (CH₃)₃Si-[O-Si(CH₃)₂]₉₇₆-O-Si(CH₃)₃, (CH₃)₃Si-[O-Si(CH₃)₂]₉₇₇-O-Si(CH₃)₃, (CH₃)₃Si-[O-Si(CH₃)₂]₉₇₈-O-Si(CH₃)₃, (CH₃)₃Si-[O-Si(CH₃)₂]₉₇₉-O-Si(CH₃)₃, (CH₃)₃Si-[O-Si(CH₃)₂]₉₈₀-O-Si(CH₃)₃, (CH₃)₃Si-[O-Si(CH₃)₂]₉₈₁-O-Si(CH₃)₃, (CH₃)₃Si-[O-Si(CH₃)₂]₉₈₂-O-Si(CH₃)₃, (CH₃)₃Si-[O-Si(CH₃)₂]₉₈₃-O-Si(CH₃)₃, (CH₃)₃Si-[O-Si(CH₃)₂]₉₈₄-O-Si(CH₃)₃, (CH₃)₃Si-[O-Si(CH₃)₂]₉₈₅-O-Si(CH₃)₃, (CH₃)₃Si-[O-Si(CH₃)₂]₉₈₆-O-Si(CH₃)₃, (CH₃)₃Si-[O-Si(CH₃)₂]₉₈₇-O-Si(CH₃)₃, (CH₃)₃Si-[O-Si(CH₃)₂]₉₈₈-O-Si(CH₃)₃, (CH₃)₃Si-[O-Si(CH₃)₂]₉₈₉-O-Si(CH₃)₃, (CH₃)₃Si-[O-Si(CH₃)₂]₉₉₀-O-Si(CH₃)₃, (CH₃)₃Si-[O-Si(CH₃)₂]₉₉₁-O-Si(CH₃)₃, (CH₃)₃Si-[O-Si(CH₃)₂]₉₉₂-O-Si(CH₃)₃, (CH₃)₃Si-[O-Si(CH₃)₂]₉₉₃-O-Si(CH₃)₃, (CH₃)₃Si-[O-Si(CH₃)₂]₉₉₄-O-Si(CH₃)₃, (CH₃)₃Si-[O-Si(CH₃)₂]₉₉₅-O-Si(CH₃)₃, (CH₃)₃Si-[O-Si(CH₃)₂]₉₉₆-O-Si(CH₃)₃, (CH₃)₃Si-[O-Si(CH₃)₂]₉₉₇-O-Si(CH₃)₃, (CH₃)₃Si-[O-Si(CH₃)₂]₉₉₈-O-Si(CH₃)₃, (CH₃)₃Si-[O-Si(CH₃)₂]₉₉₉-O-Si(CH₃)₃, (CH₃)₃Si-[O-Si(CH₃)₂]₁₀₀₀-O-Si(CH₃)₃, (CH₃)₃Si-[O-Si(CH₃)₂]₁₀₀₁-O-Si(CH₃)₃, (CH₃)₃Si-[O-Si(CH₃)₂]₁₀₀₂-O-Si(CH₃)₃, (CH₃)₃Si-[O-Si(CH₃)₂]₁₀₀₃-O-Si(CH₃)₃, (CH₃)₃Si-[O-Si(CH₃)₂]₁₀₀₄-O-Si(CH₃)₃, (CH₃)₃Si-[O-Si(CH₃)₂]₁₀₀₅-O-Si(CH₃)₃, (CH₃)₃Si-[O-Si(CH₃)₂]₁₀₀₆-O-Si(CH₃)₃, (CH₃)₃Si-[O-Si(CH₃)₂]₁₀₀₇-O-Si(CH₃)₃, (CH₃)₃Si-[O-Si(CH₃)₂]₁₀₀₈-O-Si(CH₃)₃, (CH₃)₃Si-[O-Si(CH₃)₂]₁₀₀₉-O-Si(CH₃)₃, (CH₃)₃Si-[O-Si(CH₃)₂]₁₀₁₀-O-Si(CH₃)₃, (CH₃)₃Si-[O-Si(CH₃)₂]₁₀₁₁-O-Si(CH₃)₃, (CH₃)₃Si-[O-Si(CH₃)₂]₁₀₁₂-O-Si(CH₃)₃, (CH₃)₃Si-[O-Si(CH₃)₂]₁₀₁₃-O-Si(CH₃)₃, (CH₃)₃Si-[O-Si(CH₃)₂]₁₀₁₄-O-Si(CH₃)₃, (CH₃)₃Si-[O-Si(CH₃)₂]₁₀₁₅-O-Si(CH₃)₃, (CH₃)₃Si-[O-Si(CH₃)₂]₁₀₁₆-O-Si(CH₃)₃, (CH₃)₃Si-[O-Si(CH₃)₂]₁₀₁₇-O-Si(CH₃)₃, (CH₃)₃Si-[O-Si(CH₃)₂]₁₀₁₈-O-Si(CH₃)₃, (CH₃)₃Si-[O-Si(CH₃)₂]₁₀₁₉-O-Si(CH₃)₃, (CH₃)₃Si-[O-Si(CH₃)₂]₁₀₂₀-O-Si(CH₃)₃, (CH₃)₃Si-[O-Si(CH₃)₂]₁₀₂₁-O-Si(CH₃)₃, (CH₃)₃Si-[O-Si(CH₃)₂]₁₀₂₂-O-Si(CH₃)₃, (CH₃)₃Si-[O-Si(CH₃)₂]₁₀₂₃-O-Si(CH₃)₃, (CH₃)₃Si-[O-Si(CH₃)₂]₁₀₂₄-O-Si(CH₃)₃, (CH₃)₃Si-[O-Si(CH₃)₂]₁₀₂₅-O-Si(CH₃)₃, (CH₃)₃Si-[O-Si(CH₃)₂]₁₀₂₆-O-Si(CH₃)₃, (CH₃)₃Si-[O-Si(CH₃)₂]₁₀₂₇-O-Si(CH₃)₃, (CH₃)₃Si-[O-Si(CH₃)₂]₁₀₂₈-O-Si(CH₃)₃, (CH₃)₃Si-[O-Si(CH₃)₂]₁₀₂₉-O-Si(CH₃)₃, (CH₃)₃Si-[O-Si(CH₃)₂]₁₀₃₀-O-Si(CH₃)₃, (CH₃)₃Si-[O-Si(CH₃)₂]₁₀₃₁-O-Si(CH₃)₃, (CH₃)₃Si-[O-Si(CH₃)₂]₁₀₃₂-O-Si(CH₃)₃, (CH₃)₃Si-[O-Si(CH₃)₂]₁₀₃₃-O-Si(CH₃)₃, (CH₃)₃Si-[O-Si(CH₃)₂]₁₀₃₄-O-Si(CH₃)₃, (CH₃)₃Si-[O-Si(CH₃)₂]₁₀₃₅-O-Si(CH₃)₃, (CH₃)₃Si-[O-Si(CH₃)₂]₁₀₃₆-O-Si(CH₃)₃, (CH₃)₃Si-[O-Si(CH₃)₂]₁₀₃₇-O-Si(CH₃)₃, (CH₃)₃Si-[O-Si(CH₃)₂]₁₀₃₈-O-Si(CH₃)₃, (CH₃)₃Si-[O-Si(CH₃)₂]₁₀₃₉-O-Si(CH₃)₃, (CH₃)₃Si-[O-Si(CH₃)₂]₁₀₄₀-O-Si(CH₃)₃, (CH₃)₃Si-[O-Si(CH₃)₂]₁₀₄₁-O-Si(CH₃)₃, (CH₃)₃Si-[O-Si(CH₃)₂]₁₀₄₂-O-Si(CH₃)₃, (CH₃)₃Si-[O-Si(CH₃)₂]₁₀₄₃-O-Si(CH₃)₃, (CH₃)₃Si-[O-Si(CH₃)₂]₁₀₄₄-O-Si(CH₃)₃, (CH₃)₃Si-[O-Si(CH₃)₂]₁₀₄₅-O-Si(CH₃)₃, (CH₃)₃Si-[O-Si(CH₃)₂]₁₀₄₆-O-Si(CH₃)₃, (CH₃)₃Si-[O-Si(CH₃)₂]₁₀₄₇-O-Si(CH₃)₃, (CH₃)₃Si-[O-Si(CH₃)₂]₁₀₄₈-O-Si(CH₃)₃, (CH₃)₃Si-[O-Si(CH₃)₂]₁₀₄₉-O-Si(CH₃)₃, (CH₃)₃Si-[O-Si(CH₃)₂]₁₀₅₀-O-Si(CH₃)₃, (CH₃)₃Si-[O-Si(CH₃)₂]₁₀₅₁-O-Si(CH₃)₃, (CH₃)₃Si-[O-Si(CH₃)₂]₁₀₅₂-O-Si(CH₃)₃, (CH₃)₃Si-[O-Si(CH₃)₂]₁₀₅₃-O-Si(CH₃)₃, (CH₃)₃Si-[O-Si(CH₃)₂]₁₀₅₄-O-Si(CH₃)₃, (CH₃)₃Si-[O-Si(CH₃)₂]₁₀₅₅-O-Si(CH₃)₃, (CH₃)₃Si-[O-Si(CH₃)₂]₁₀₅₆-O-Si(CH₃)₃, (CH₃)₃Si-[O-Si(CH₃)₂]₁₀₅₇-O-Si(CH₃)₃, (CH₃)₃Si-[O-Si(CH₃)₂]₁₀₅₈-O-Si(CH₃)₃, (CH₃)₃Si-[O-Si(CH₃)₂]₁₀₅₉-O-Si(CH₃)₃, (CH₃)₃Si-[O-Si(CH₃)₂]₁₀₆₀-O-Si(CH₃)₃, (CH₃)₃Si-[O-Si(CH₃)₂]₁₀₆₁-O-Si(CH₃)₃, (CH₃)₃Si-[O-Si(CH₃)₂]₁₀₆₂-O-Si(CH₃)₃, (CH₃)₃Si-[O-Si(CH₃)₂]₁₀₆₃-O-Si(CH₃)₃, (CH₃)₃Si-[O-Si(CH₃)₂]₁₀₆₄-O-Si(CH₃)₃, (CH₃)₃Si-[O-Si(CH₃)₂]₁₀₆₅-O-Si(CH₃)₃, (CH₃)₃Si-[O-Si(CH₃)₂]₁₀₆₆-O-Si(CH₃)₃, (CH₃)₃Si-[O-Si(CH₃)₂]₁₀₆₇-O-Si(CH₃)₃, (CH₃)₃Si-[O-Si(CH₃)₂]₁₀₆₈-O-Si(CH₃)₃, (CH₃)₃Si-[O-Si(CH₃)₂]₁₀₆₉-O-Si(CH₃)₃, (CH₃)₃Si-[O-Si(CH₃)₂]₁₀₇₀-O-Si(CH₃)₃, (CH₃)₃Si-[O-Si(CH₃)₂]₁₀₇₁-O-Si(CH₃)₃, (CH₃)₃Si-[O-Si(CH₃)₂]₁₀₇₂-O-Si(CH₃)₃, (CH₃)₃Si-[O-Si(CH₃)₂]₁₀₇₃-O-Si(CH₃)₃, (CH₃)₃Si-[O-Si(CH₃)₂]₁₀₇₄-O-Si(CH₃)₃, (CH₃)₃Si-[O-Si(CH₃)₂]₁₀₇₅-O-Si(CH₃)₃, (CH₃)₃Si-[O-Si(CH₃)₂]₁₀₇₆-O-Si(CH₃)₃, (CH₃)₃Si-[O-Si(CH₃)₂]₁₀₇₇-O-Si(CH₃)₃, (CH₃)₃Si-[O-Si(CH₃)₂]₁₀₇₈-O-Si(CH₃)₃, (CH₃)₃Si-[O-Si(CH₃)₂]₁₀₇₉-O-Si(CH₃)₃, (CH₃)₃Si-[O-Si(CH₃)₂]₁₀₈₀-O-Si(CH₃)₃, (CH₃)₃Si-[O-Si(CH₃)₂]₁₀₈₁-O-Si(CH₃)₃, (CH₃)₃Si-[O-Si(CH₃)₂]₁₀₈₂-O-Si(CH₃)₃, (CH₃)₃Si-[O-Si(CH₃)₂]₁₀₈₃-O-Si(CH₃)₃, (CH₃)₃Si-[O-Si(CH₃)₂]₁₀₈₄-O-Si(CH₃)₃, (CH₃)₃Si-[O-Si(CH₃)₂]₁₀₈₅-O-Si(CH₃)₃, (CH₃)₃Si-[O-Si(CH₃)₂]₁₀₈₆-O-Si(CH₃)₃, (CH₃)₃Si-[O-Si(CH₃)₂]₁₀₈₇-O-Si(CH₃)₃, (CH₃)₃Si-[O-Si(CH₃)₂]₁₀₈₈-O-Si(CH₃)₃, (CH₃)₃Si-[O-Si(CH₃)₂]₁₀₈₉-O-Si(CH₃)₃, (CH₃)₃Si-[O-Si(CH₃)₂]₁₀₉₀-O-Si(CH₃)₃, (CH₃)₃Si-[O-Si(CH₃)₂]₁₀₉₁-O-Si(CH₃)₃, (CH₃)₃Si-[O-Si(CH₃)₂]₁₀₉₂-O-Si(CH₃)₃, (CH₃)₃Si-[O-Si(CH₃)₂]₁₀₉₃-O-Si(CH₃)₃, (CH₃)₃Si-[O-Si(CH₃)₂]₁₀₉₄-O-Si(CH₃)₃, (CH₃)₃Si-[O-Si(CH₃)₂]₁₀₉₅-O-Si(CH₃)₃, (CH₃)₃Si-[O-Si(CH₃)₂]₁₀₉₆-O-Si(CH₃)₃, (CH₃)₃Si-[O-Si(CH₃)₂]₁₀₉₇-O-Si(CH₃)₃, (CH₃)₃Si-[O-Si(CH₃)₂]₁₀₉₈-O-Si(CH₃)₃, (CH₃)₃Si-[O-Si(CH₃)₂]₁₀₉₉-O-Si(CH₃)₃, (CH₃)₃Si-[O-Si(CH₃)₂]₁₁₀₀-O-Si(CH₃)₃, (CH₃)₃Si-[O-Si(CH₃)₂]-O-Si(CH₃)₃ sowie Mischungen dieser Substanzen.

Als zweiten wesentlichen Bestandteil enthalten die erfindungsgemäßen Mittel mindestens ein Silikon der Formel Si-II

**(CH₃)₃Si-[O-Si(CH₃)₂]ₓ-[O-(H₃C)Si((CH₂)ₖ(CH₂CH₂O)ₘ( CH₂CH₂CH₂O)ₙ)]ₓ-O-Si(CH₃)₃** **(Si-II),**

in der
- k für Werte von 1 bis 20, vorzugsweise von 2 bis 10 und insbesondere für 2, 3, 4, 5, 6 steht;
- m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
- n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
- x bzw. y jeweils unabhängig voneinander für eine Zahl von 0 bis 5000, vorzugsweise von 10 bis 2500, weiter bevorzugt von 50 bis 1500 und insbesondere 100 bis 1000 stehen,
mit der Maßgabe, daß (m + n) nicht Null ist.

Bei diesen Silikone ist eine Methylgruppe einiger Dimethylsiloxan-Replizierungseinheiten durch eine ethoxylierte und/oder propoxylierte Alkylengruppe ersetzt, wodurch diese Silikone wasserlöslich sind. Nach der INCI-Nomenklatur werden Silikone der Formel Si-II als Dimethicone Copolyole bezeichnet.

Erfindungsgemäß bevorzugte Mittel enthalten Dimethicone Copolyole, d.h. Silikone der Formel Si-II, in engeren Mengen. Hier sind erfindungsgemäße kosmetische Mittel bevorzugt, die - bezogen auf ihr Gewicht - 0,02 bis 8,5 Gew.-%, vorzugsweise 0,1 bis 7,5 Gew.-%, besonders bevorzugt 0,25 bis 5 Gew.-%, weiter bevorzugt 0,25 bis 4 Gew.-% und insbesondere 0,3 bis 2,5 Gew.-% mindestens eines Silikons der Formel Si-II enthalten.

Auch die Silikone der Formel Si-II können Oligomeriserungs- bzw. Polymerisationsgrade zwischen 0 und 5000 aufweisen, wobei bevorzugte Oligomeriserungs- bzw. Polymerisationsgrade im Bereich von 10 bis 2500, weiter bevorzugt von 50 bis 1500 und insbesondere von 100 bis 1000 liegen. Naturgemäß variiert das Molekulargewicht der Dimethicone Copolyole ebenfalls mit dem Oligomeriserungs- bzw. Polymerisationsgrad.

Erfindungsgemäß in den Mitteln enthaltene Dimethicone Copolyole weisen die Polimersisierungsgrade x (für die unsubstituierte Dimethylsiloxan-Einheit) bzw. y (für die substituierte Dimethylsiloxan-Einheit) auf. Obwohl gemäß Definition von x und y theoretisch "Gesamt"-Polymerisationsgrade (Summe x + y) von bis zu 10.000 möglich sind, sind erfindungsgemäße Mittel bevorzugt, bei denen die Summe x + y im Bereich von 10 bis 2500, weiter bevorzugt von 50 bis 1500 und insbesondere von 100 bis 1000 liegt. Die Darstellung in Formel Si-II ist schematisch. Sie bedeutet nicht, daß die substituierten und unsubstituierten Dimethylsiloxan-Einheiten blockweise vorliegen müssen; vielmehr können die -[O-Si(CH₃)₂]ₓ-Einheiten und die -[O-(H₃C)Si((CH₂)ₖ(CH₂CH₂O)ₘ(CH₂CH₂CH₂O)ₙ)]_{y}-Einheiten auch "randomized", d.h. statistisch über das Molekül verteilt vorliegen.

Die Viskosität der erfindungsgemäß eingesetzten Dimethicone Copolyole ist ebenfalls nahezu temperaturunabhängig und variiert mit der Molmasse, wobei hochmolekulare Dimethicone Copolyole höhere Viskositäten aufweisen als niedermolekulare. Hier sind erfindungsgemäße kosmetische Mittel bevorzugt, die als Silikon der Formel Si-II ein Dimethicone Copolyol enthalten, das eine Viskosität von 1 bis 10.000 cSt, vorzugsweise von 10 bis 5000 cSt, besonders bevorzugt von 50 bis 2500 cSt, weiter bevorzugt von 100 bis 1000 cSt und insbesondere von 300 bis 500 cSt (gemessen bei 20°C) aufweist.

Erfindungsgemäß beträgt der Ethoxylierungsgrad m 0 bis 100, vorzugsweise 2 bis 50, besonders bevorzugt 5 bis 35 und insbesondere 8, 9, 10, 11, 12, 13, 14, 15, 16, 17, 18, 19, 20. Der Propoxylierungsgrad n steht für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20. Auch hier bedeutet die formelmäßige Darstellung in der Formel Si-II nicht, daß die Ethylenoxideinheiten zwingend an die Alkylengruppe gebunden sein müssen, die mit dem Si-Atom verbunden ist. Vielmehr können EO- und PO-Einheiten auch vertauscht vorliegen, so daß eine -[O-(H₃C)Si((CH₂)ₖ(CH₂CH₂CH₂O)ₘ(CH₂CH₂O)ₙ)]_{y}-Einheit ebenfalls möglich ist. Auch hier bedeutet die Abfolge EOₘPOₙ nicht zwingend, daß die EO- bzw. PO-Einheiten blockweise vorliegen müssen; vielmehr können die EO- und PO-Einheiten auch "randomized", d.h. statistisch über das Molekül verteilt vorliegen.

Die Alkylengruppe in Formel Si-II ist vorzugsweise eine lineare Alkylengruppe, bei der k für Werte von 1 bis 20, vorzugsweise von 2 bis 10 und insbesondere für 2, 3, 4, 5, 6 steht. Bevorzugt sind daher die Gruppierungen -CH₂-, -CH₂-CH₂-, -CH₂-CH₂-CH₂-, -CH₂-CH₂-CH₂-CH₂-, -CH₂-CH₂-CH₂-CH₂-CH₂-, -CH₂-CH₂-CH₂-CH₂-CH₂-CH₂-CH₂-CH₂-CH₂-CH₂-.

Erfindungsgemäß besonders bevorzugte Dimehicon-Copolyole sind im Prioritätsdokument auf den Seiten 10 bis 140 explizit aufgeführt. Auf die Offenbarung dieser Gruppe besonders bevozugter Dimehicon-Copolyole im Prioritätsdokument wird hier ausdrücklich Bezug genommen.

Vorzugsweise werden die Silikone der Formel Si-I und die Silikone der Formel Si-II zueinander in bestimmten Gewichtsverhältnissen eingesetzt. Hier sind erfindungsgemäße kosmetische Mittel bevorzugt, bei denen das Gewichtsverhältnis der im Mittel enthaltenen Silikone der Formel Si-I zu den im Mittel enthaltenen Silikonen der Formel Si-II 10:1 bis 1:10, vorzugsweise 8:1 bis 1:8, besonders bevorzugt 7:1 bis 1:6, weiter bevorzugt 4:1 bis 1:3 und insbesondere 3:1 bis 1:2 beträgt.

Als weiteren optionalen Bestandteil können die erfindungsgemäßen Mittel 0,01 bis 10 Gew.-% mindestens eines Polymers aus der Gruppe der kationischen und/oder amphoteren Polymere enthalten.

Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet.

Vorzugsweise wird das Polymer bzw. werden die Polymere innerhalb engerer Mengenbereiche eingesetzt. So sind erfindungsgemäße Mittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% amphotere(s) Polymer(e), enthalten.

Unabhängig davon, ob in den Mitteln amphotere Polymere enthalten sind oder nicht, sind weiter bevorzugte erfindungsgemäße Mittel dadurch gekennzeichnet, daß sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten.

### Erfindungsgemäß bevorzugt einsetzbare kationische Polymere werden nachstehend beschrieben:

Homopolymere der allgemeinen Formel (G1-I), 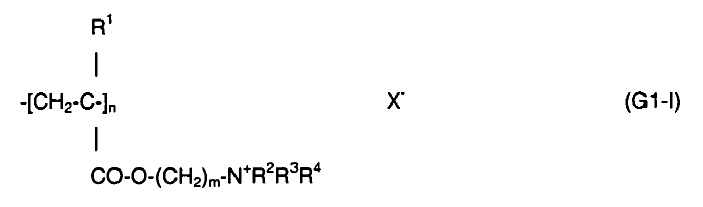 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCl-Bezeichnung: PPG-1-Trideceth-6)) im Handel erhältlich.

Copolymere mit Monomereinheiten gemäß Formel (G1-1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Als kationische Polymere können auch kationische Proteinhydrolysate eingesetzt werden, wobei bevorzugte Mittel ein oder mehrere kationische Proteinhydrolysate aus der Gruppe Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein enthalten.

Zusammenfassend sind erfindungsgemäße kosmetische Mittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten, wobei bevorzugte kationische(s) Polymer(e) ausgewählt ist/sind aus
a. Poly(methacryloyloxyethyltrimethylammoniumchlorid) (INCl: Polyquaternium-37) und/oder;
b. quaternisierten Cellulose-Derivaten (INCI: Polyquaternium 10) und/oder
c. kationischen Alkylpolyglycosiden und/oder
d. kationisertem Honig und/oder
e. kationischen Guar-Derivaten und/oder
f. polymeren Dimethyldiallylammoniumsalzen und deren Copolymeren mit Estern und Amiden von Acrylsäure und Methacrylsäure und/oder
g. Copolymeren des Vinylpyrrolidons mit quaternierten Derivaten des Dialkylaminoalkylacrylats und -methacrylats und/oder
h. Vinylpyrrolidon-Vinylimidazoliummethochlorid-Copolymeren und/oder
i. quaterniertem Polyvinylalkohol und/oder
j. Polyquaternium 2 und/oder
k. Polyquaternium-7 und/oder
l. Polyquaternium 17 und/oder
m. Polyquaternium 18 und/oder
n. Polyquaternium 24 und/oder
o. Polyquaternium 27.

Zusätzlich zu den kationischen Polymeren oder an ihrer Stelle können die erfindungsgemäßen Mittel amphotere Polymere enthalten. Im Rahmen der vorliegenden Erfindung bevorzugt einsetzbare amphotere Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I), die Derivate des Acrylamids oder Methacrylamids darstellen. Weiterhin bevorzugt sind solche Monomeren, die als Gegenionen Halogenid-, Methoxysulfat- oder Ethoxysulfat-lonen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Erfindungsgemäß bevorzugte Mittel sind dadurch gekennzeichnet, daß das/die amphotere(n) Polymer(e) Monomere A) und B) umfassen, wobei A) und B) ausgewählt sind aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in der R⁶ und R⁷ unabhängig voneinander Wasserstoff oder Methylgruppen sind.

Mit besonderem Vorzug enthalten die in den erfindungsgemäßen Mitteln eingesetzten amphoteren Polymere Monomere aus der Gruppe der Acrylamide und/oder Methacrylamide mit Alkylammoniumgruppen. Als zusätzlich in den Polymeren enthaltene Monomere mit anionischen Gruppen haben sich Acrylsäure und/oder Methacrylsäure und/oder Crotonsäure und/oder 2-Methyl-crotonsäure bewährt.

Zusammenfassend sind erfindungsgemäße Mittel bevorzugt, bei denen das/die amphotere(n) Polymer(e) Co-Polymerisate mindestens eines der Monomere
- Trimethylammoniumethylacrylamid und/oder,
- Trimethylammoniumethylmethacrylamid und/oder
- Trimethylammoniumpropylacrylamid und/oder
- Trimethylammoniumpropylmethacrylamid und/oder
- Trimethylammoniumethylacrylamid und/oder
- Trimethylammoniumethylacrylat und/oder
- Trimethylammoniumethylmethacrylat und/oder
- Trimethylammoniumethylacrylat und/oder
- Ethyldimethylammoniumethylacrylamid und/oder,
- Ethyldimethylammoniumethylmethacrylamid und/oder
- Ethyldimethylammoniumpropylacrylamid und/oder
- Ethyldimethylammoniumpropylmethacrylamid und/oder
- Ethyldimethylammoniumethylacrylamid und/oder
- Ethyldimethylammoniumethylacrylat und/oder
- Ethyldimethylammoniumethylmethacrylat und/oder
- Ethyldimethylammoniumethylacrylat
mit mindetsnes einem der Monomere
- Acrylsäure und/oder
- Methacrylsäure und/oder
- Crotonsäure und/oder
- 2-Methyl-crotonsäure
sind.

Erfindungsgemäß besonders bevorzugte amphotere Polymere sind:
- Copolymere von Trimethylammoniumethylacrylamid mit Acrylsäure
- Copolymere von Trimethylammoniumethylacrylamid mit Methacrylsäure
- Copolymere von Trimethylammoniumethylacrylamid mit Crotonsäure
- Copolymere von Trimethylammoniumethylacrylamid mit 2-Methyl-crotonsäure
- Copolymere von Trimethylammoniumethylmethacrylamid mit Acrylsäure
- Copolymere von Trimethylammoniumethylmethacrylamid mit Methacrylsäure
- Copolymere von Trimethylammoniumethylmethacrylamid mit Crotonsäure
- Copolymere von Trimethylammoniumethylmethacrylamid mit 2-Methyl-crotonsäure
- Copolymere von Trimethylammoniumpropylacrylamid mit Acrylsäure
- Copolymere von Trimethylammoniumpropylacrylamid mit Methacrylsäure
- Copolymere von Trimethylammoniumpropylacrylamid mit Crotonsäure
- Copolymere von Trimethylammoniumpropylacrylamid mit 2-Methyl-crotonsäure
- Copolymere von Trimethylammoniumpropylmethacrylamid mit Acrylsäure
- Copolymere von Trimethylammoniumpropylmethacrylamid mit Methacrylsäure
- Copolymere von Trimethylammoniumpropylmethacrylamid mit Crotonsäure
- Copolymere von Trimethylammoniumpropylmethacrylamid mit 2-Methyl-crotonsäure
- Copolymere von Trimethylammoniumethylacrylamid mit Acrylsäure
- Copolymere von Trimethylammoniumethylacrylamid mit Methacrylsäure
- Copolymere von Trimethylammoniumethylacrylamid mit Crotonsäure
- Copolymere von Trimethylammoniumethylacrylamid mit 2-Methyl-crotonsäure
- Copolymere von Trimethylammoniumethylacrylat mit Acrylsäure
- Copolymere von Trimethylammoniumethylacrylat mit Methacrylsäure
- Copolymere von Trimethylammoniumethylacrylat mit Crotonsäure
- Copolymere von Trimethylammoniumethylacrylat mit 2-Methyl-crotonsäure
- Copolymere von Trimethylammoniumethylmethacrylat mit Acrylsäure
- Copolymere von Trimethylammoniumethylmethacrylat mit Methacrylsäure
- Copolymere von Trimethylammoniumethylmethacrylat mit Crotonsäure
- Copolymere von Trimethylammoniumethylmethacrylat mit 2-Methyl-crotonsäure
- Copolymere von Trimethylammoniumethylacrylat mit Acrylsäure
- Copolymere von Trimethylammoniumethylacrylat mit Methacrylsäure
- Copolymere von Trimethylammoniumethylacrylat mit Crotonsäure
- Copolymere von Trimethylammoniumethylacrylat mit 2-Methyl-crotonsäure
- Copolymere von Ethyldimethylammoniumethylacrylamid mit Acrylsäure
- Copolymere von Ethyldimethylammoniumethylacrylamid mit Methacrylsäure
- Copolymere von Ethyldimethylammoniumethylacrylamid mit Crotonsäure
- Copolymere von Ethyldimethylammoniumethylacrylamid mit 2-Methyl-crotonsäure
- Copolymere von Ethyldimethylammoniumethylmethacrylamid mit Acrylsäure
- Copolymere von Ethyldimethylammoniumethylmethacrylamid mit Methacrylsäure
- Copolymere von Ethyldimethylammoniumethylmethacrylamid mit Crotonsäure
- Copolymere von Ethyldimethylammoniumethylmethacrylamid mit 2-Methyl-crotonsäure
- Copolymere von Ethyldimethylammoniumpropylacrylamid mit Acrylsäure
- Copolymere von Ethyldimethylammoniumpropylacrylamid mit Methacrylsäure
- Copolymere von Ethyldimethylammoniumpropylacrylamid mit Crotonsäure
- Copolymere von Ethyldimethylammoniumpropylacrylamid mit 2-Methyl-crotonsäure
- Copolymere von Ethyldimethylammoniumpropylmethacrylamid mit Acrylsäure
- Copolymere von Ethyldimethylammoniumpropylmethacrylamid mit Methacrylsäure
- Copolymere von Ethyldimethylammoniumpropylmethacrylamid mit Crotonsäure
- Copolymere von Ethyldimethylammoniumpropylmethacrylamid mit 2-Methyl-crotonsäure
- Copolymere von Ethyldimethylammoniumethylacrylamid mit Acrylsäure
- Copolymere von Ethyldimethylammoniumethylacrylamid mit Methacrylsäure
- Copolymere von Ethyldimethylammoniumethylacrylamid mit Crotonsäure
- Copolymere von Ethyldimethylammoniumethylacrylamid mit 2-Methyl-crotonsäure
- Copolymere von Ethyldimethylammoniumethylacrylat mit Acrylsäure
- Copolymere von Ethyldimethylammoniumethylacrylat mit Methacrylsäure
- Copolymere von Ethyldimethylammoniumethylacrylat mit Crotonsäure
- Copolymere von Ethyldimethylammoniumethylacrylat mit 2-Methyl-crotonsäure
- Copolymere von Ethyldimethylammoniumethylmethacrylat mit Acrylsäure
- Copolymere von Ethyldimethylammoniumethylmethacrylat mit Methacrylsäure
- Copolymere von Ethyldimethylammoniumethylmethacrylat mit Crotonsäure
- Copolymere von Ethyldimethylammoniumethylmethacrylat mit 2-Methyl-crotonsäure
- Copolymere von Ethyldimethylammoniumethylacrylat mit Acrylsäure
- Copolymere von Ethyldimethylammoniumethylacrylat mit Methacrylsäure
- Copolymere von Ethyldimethylammoniumethylacrylat mit Crotonsäure
- Copolymere von Ethyldimethylammoniumethylacrylat mit 2-Methyl-crotonsäure

Vorzugsweise enthalten die erfindungsgemäßen Mittel zusätzlich mindestens einen Emulgator bzw. ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 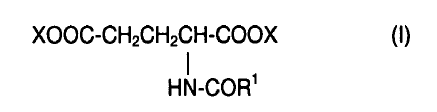 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 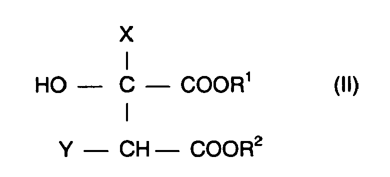 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 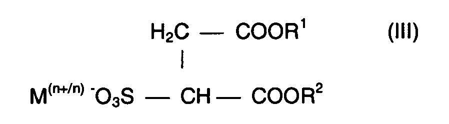 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.
Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®} - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht.

Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov^{®}68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls^{®} PGPH) oder Triglycerindiisostearat (Lameform^{®} TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Ganz besonders bevorzugt sind erfindungsgemäße Mittel, die zusätzlich Fettalkohol(e) und/oder Fettalkoholalkoxylat(e), vorzugsweise C₁₂₋₂₂-Fettalkohol(e) und/oder C₁₂₋₂₂-Fettalkoholethoxylat(e) mit 10 bis 30 EO-Einheiten, besonders bevorzugt C₁₆₋₁₈-Fettalkohol(e) und/oder C₁₆₋₁₈-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht des Mittels, enthalten.

Zusammenfassend sind erfindungsgemäße kosmetische Mittel bevorzugt, die zusätzlich Tensid(e), vorzugsweise anionische(s) Tensid(e), besonders bevorzugt Fettalkoholethersulfate der Formel

**H₃C-(CH₂)ₙ(OCH₂CH₂)ₖ-OSO₃⁻M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4 oder 4, vorzugsweise für 2, und M für ein Kation aus der Gruppe Na⁺, K⁺ NH₄⁺, ½ Mg²⁺, ½ Zn²⁺, vorzugsweise für Na⁺, stehen,
wobei bevorzugte Mittel das bzw. die Tensid(e) in Mengen von 2 bis 20 Gew.-%, vorzugsweise von 5 bis 15 Gew.-% und insbesondere von 7,5 bis 12 Gew.-%, jeweils bezogen auf das gesamte Mittel, enthalten.

Bevorzugte erfindungsgemäße kosmetische Mittel sind dadurch gekennzeichnet, daß sie zusätzlich amphotere(s) Tensid(e), vorzugsweise aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betaine bekannten Verbindungen,
- der unter der INCI-Bezeichnung Coco-Betaine bekannten Verbindungen,
- der unter der INCI-Bezeichnung Disodium Cocoamphodiacetate bekannten Verbindungen,
enthalten, wobei bevorzugte Mittel das bzw. die amphotere(n) Tensid(e) in Mengen von 1 bis 15 Gew.-%, vorzugsweise von 2,5 bis 12 Gew.-% und insbesondere von 5 bis 10 Gew.-%, jeweils bezogen auf das gesamte Mittel, enthalten.

Als weiteren Inhaltsstoff können die erfindungsgemäßen Mittel Purin und/oder Derivat(e) des Purins enthalten. Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Mittel enthalten Purin und/oder Purinderivate in bestimmten Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte kosmetische Mittel sind dadurch gekennzeichnet, daß sie Purin und/oder Purinderivat(e) der Formel (I) enthalten 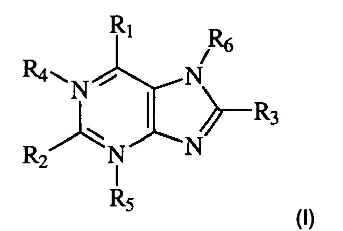 in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = H)
- Theophyllin (R¹ = R² = OH, R³ = H, R⁴ = CH₃, R⁵ = CH₃, R⁶ = H)

Je nach gewünschtem Anwendungszweck der kosmetischen Mittel kann dabei die Art und Menge des Purinderivates variieren. In haarkosmetischen Formulierungen hat sich insbesondere Coffein bewährt, das beispielsweise in Shampoos vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf das Shampoo) eingesetzt werden kann.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte kosmetische Mittel dadurch gekennzeichnet, daß sie 0,05 bis 4,5 Gew.-%, vorzugsweise 0,1 bis 4 Gew.-%, besonders bevorzugt 0,5 bis 3,5 Gew.-% und insbesondere 0,75 bis 2,5 Gew.-% Kohlenhydrat(e) enthalten, wobei bevorzugte Kohlenhydrate ausgewählt sind aus
- Monosachhariden, insbesondere
- D-Ribose und/oder
- D-Xylose und/oder
- L-Arabinose und/oder
- D-Glucose und/oder
- D-Mannose und/oder
- D-Galactose und/oder
- D-Fructose und/oder
- Sorbose und/oder
- L-Fucose und/oder
- L-Rhamnose
- Disacchariden, insbesondere
- Saccharose und/oder
- Maltose und/oder
- Lactose und/oder
- Trehalose und/oder
- Cellobiose und/oder
- Gentiobiose und/oder
- Isomaltose.

Besonders bevorzugte erfindungsgemäße Mittel enthalten zusätzlich zu Kohlenhydrat(en) die weiter oben erwähnten Purine bzw. Purinderivate. Insbesondere bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-HydroxyL-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte erfindungsgemäße Mittel enthalten zusätzlich zu Aminosäure(n) die weiter oben erwähnten Purine bzw. Purinderivate. Insbesondere bevorzugte erfindungsgemäße Mittel enthalten bezogen auf ihr Gewicht
- 0,005 bis 0,015 Gew.-% Coffein und 0,1 bis 0,25 Gew.-% Glycin,
- 0,005 bis 0,015 Gew.-% Coffein und 0,1 bis 0,25 Gew.-% Alanin,
- 0,005 bis 0,015 Gew.-% Coffein und 0,1 bis 0,25 Gew.-% Valin.

Zusätzlich können die erfindungsgemäßen Mittel 0,01 bis 5 Gew.-% Hydantoin bzw. mindestens eines Hydatoinderivates enthalten. Besonders bevorzugt werden erfindungsgemäß Hydantoinderivate eingesetzt, wobei 5-Ureidohydantoin besonders bevorzugt ist. Unabhängig davon, ob Hydantoin oder Hydantoinderivat(e) eingesetzt wird/werden, sind Einsatzmengen von 0,02 bis 2,5 Gew.-%, bevorzugt von 0,05 bis 1,5 Gew.-%, weiter bevorzugt 0,075 bis 1 Gew.-% und insbesondere 0,1 bis 0,25 Gew.-% - jeweils bezogen auf das gesamte Mittel - bevorzugt.

Zusammenfassend sind erfindungsgemäße kosmetische Mittel bevorzugt, die 0,02 bis 2,5 Gew.-%, vorzugsweise 0,05 bis 1,5 Gew.-%, besonders bevorzugt 0,075 bis 1 Gew.-% und insbesondere 0,1 bis 0,25 Gew.-% Hydantoin und/oder Hydantoinderivat(e), vorzugsweise 5-Ureidohydantoin (Allantoin) 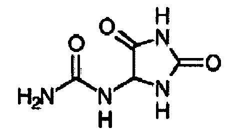 enthalten.

Besonders bevorzugte erfindungsgemäße Mittel enthalten zusätzlich zu Hydantoin und/oder Hydantoinderivat(en) die weiter oben erwähnten Purine bzw. Purinderivate. Insbesondere bevorzugte erfindungsgemäße Mittel enthalten bezogen auf ihr Gewicht 0,005 bis 0,015 Gew.-% Coffein und 0,1 bis 0,25 Gew.-% Allantoin.

Weiter bevorzugte erfindungsgemäße Mittel enthalten zusätzlich mindestens eine Aminosäure. Besonders bevorzugte erfindungsgemäße Mittel dieser Ausführungsform enthalten bezogen auf ihr Gewicht
- 0,005 bis 0,015 Gew.-% Coffein und 0,1 bis 0,25 Gew.-% Allantoin und 0,1 bis 0,25 Gew.-% Glycin,
- 0,005 bis 0,015 Gew.-% Coffein und 0,1 bis 0,25 Gew.-% Allantoin und 0,1 bis 0,25 Gew.-% Alanin,
- 0,005 bis 0,015 Gew.-% Coffein und 0,1 bis 0,25 Gew.-% Allantoin und 0,1 bis 0,25 Gew.-% Valin.

Eine weitere bevorzugte Gruppe von Inhaltsstoffen der erfindungsgemäßen Mittel sind Vitamine, Provitamine oder Vitaminvorstufen. Diese werden nachfolgend beschrieben:

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße kosmetische Mittel bevorzugt, die zusätzlich Vitamine und/oder Pro-Vitamine und/oder Vitaminvorstufen enthalten, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

Erfindungsgemäß bevorzugte Mittel enthalten zusätzlich mindestens ein 2-Furanonderivat der Formel (I) und/oder der Formel (II). 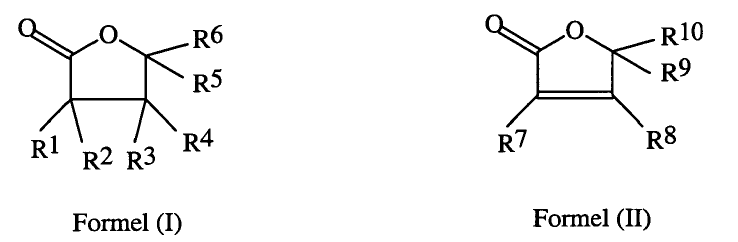 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄- gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂- C₄- gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

2-Furanone sind bekannte Verbindungen und werden beispielsweise in "Römpps Lexikon der Chemie, Interactive CD-Rom Version 2.0, zum Stichwort "Dihydro-3-hydroxy-4,4-dimethyl-2(3H)-furanon" sowie in "Ullmann's Encyclopedia, sixth edition 1999, electronic release" in den Abschnitten 2.4, 2.7, 3.2, 3.4, 4.3, 6., 11. und 15. und den dort zitierten Schriften bezüglich der Herstellung und Verwendung beschrieben. Auf diese Kapitel und die dort zitierte Literatur wird ausdrücklich Bezug genommen. Die Verbindungen der Formeln (I) und (II) werden als Zwischenstufen in der Naturstoffsynthese sowie der Herstellung von Arzneimitteln und Vitaminen eingesetzt. Die Herstellung der Wirkstoffe gemäß der Formeln (I) und (II) kann beispielsweise durch Umsetzung von primären Alkoholen mit Acrylsäuren erfolgen. Weiterhin gelangt man zu Verbindungen der Formel (I) durch Reaktionen ausgehend von Hydroxypivaldehyd. Ebenfalls führen Carbonylierungen von Alkynen zu substituierten 2-Furanonen der Formel (I) oder (II). Schließlich können die Verbindungen der Formel (I) oder der Formel (II) durch intramolekulare Veresterung der entsprechenden Hydroxycarbonsäuren erhalten werden. Beispielsweise werden die folgenden Verbindungen auf einem der zuvor aufgezeigten Synthesewege erhalten: 2,5-Dihydro-5-methoxy-2-furanon,
Tetrahydro-5-oxo-2-furancarbonsäure, Dihydro-3-hydroxy-4,4-dimethyl-2(3H)-furanon, oder 3,4-Dimethyl-5-pentylidenedihydro-2(5H)-furanon oder 4-Hydroxy-2,5-dimethyl-3(2H)-furanon. Die erfindungsgemäßen 2-Furanone umfassen selbstverständlich alle möglichen Stereoisomere wie auch deren Gemische. Durch die erfindungsgemäßen 2-Furanone wird der Geruch der kosmetischen Mittel nicht nachhaltig beeinflußt, so daß eine Parfümierung der Mittel separat erfolgen muß.

Bevorzugte Verbindungen der Formel (I) und/oder der Formel (II) können Verbindungen sein, bei welchen die Substituenten R¹, R² und R⁷ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- einen -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxyalkylrest, oder einen -C₂ - C₃₀ - gesättigten oder ein- oder mehrfach ungesättigten, verzweigten oder linearen Mono-, Di-, Tri- oder Polyaminokohlenwasserstoffrest.

In einer weiteren Ausführungsform der erfindungsgemäßen Lehre hat es sich gezeigt, daß bei den Verbindungen der Formel (I) oder der Formel (II) die Reste R³, R⁴ und R⁸ bevorzugt unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.

Weiterhin kann es bevorzugt sein, wenn in dem erfindungsgemäßen Wirkstoff gemäß der Formel(I) und/oder der Formel(II) für die Reste R⁵, R⁶, R⁹ und R¹⁰ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.

In einer besonders bevorzugten Ausführungsform der erfindungsgemäßen Lehre wird eine Verbindung der Formel (I) eingesetzt. Dabei kann es bevorzugt sein, daß in einer Verbindung der Formel (I) die Reste R¹ und R² unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxykohlenwasserstoffrest.

Weiterhin kann es in dieser besonders bevorzugten Ausführungsform der erfindungsgemäßen Lehre vorteilhaft sein, wenn in den Verbindungen der Formel (I) die Reste R³ und R⁴ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- und/oder Polyhydroxykohlenwasserstoffrest.

In dieser bevorzugten Ausführungsform kann es weiterhin vorteilhaft sein, daß die Verbindungen gemäß Formel (I) für die Reste R5 und R6 unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest.

In einer besonders bevorzugten Ausführungsform der erfindungsgemäßen Lehre wird als Verbindung entsprechend der Formel (I)
- (R)-(-)-4-Hydroxymethyl-γ-butyrolacton und/oder
- D,L-4-Hydroxymethyl-γ-butyrolacton und/oder
- (S)-(+)-4-Hydroxymethyl-γ-butyrolacton und/oder
- R-(-)-2-Hydroxy-3,3-dimethyl-γ-butyrolacton und/oder
- D,L-2-Hydroxy-3,3-dimethyl-γ-butyrolacton und/oder
- S(+)-2-Hydroxy-3,3-dimethyl-γ-butyrolacton und/oder
- 4-Hydroxy-2,5-dimethyl-3(2H)-furanon und/oder
- Tetrahydro-5-oxo-2-furancarbonsäure und/oder
- Tetrahydro-5-oxo-2-furancarbonsäure, Na-Salz und/oder
- Tetrahydro-5-oxo-2-furancarbonsäure, K-Salz und/oder
- 2,5-Dihydro-5-methoxy-2-furanon und/oder
- Dihydro-3-hydroxy-4,4-dimethyl-2(3*H*)-furanon
eingesetzt. In einer ganz besonders bevorzugten Ausführungsform der erfindungsgemäßen Lehre wird als Verbindung entsprechend der Formel (I) Dihydro-3-hydroxy-4,4-dimethyl-2(3H)-furanon eingesetzt.

Zusammenfassend sind bevorzugte erfindungsgemäße kosmetische Mittel dadurch gekennzeichnet, daß sie zusätzlich 0,05 bis 15 Gew.-% mindestens eines 2-Furanonderivats der Formel (I) und/oder der Formel (II) 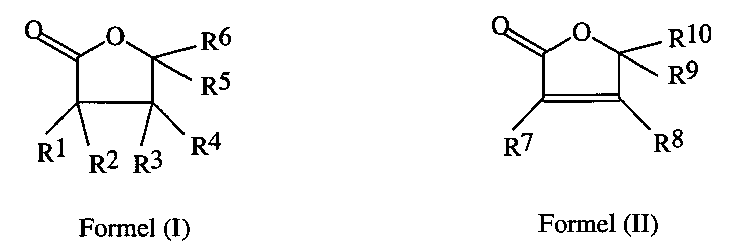 enthalten, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂- C₄- gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

Bevorzugt ist auch der zusätzlich Einsatz von Bisabolol und/oder Bisabololoxiden in den erfindungsgemäßen Mitteln. Hier sind erfindungsgemäße kosmetische Mittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 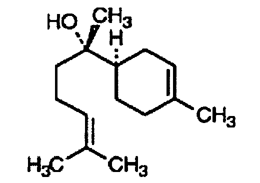 enthalten.

Die erfindungsgemäßen kosmetischen Mittel können weiterhin alle für solche Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Diese Tenside wurden weiter oben ausführlich beschrieben.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10-15 können erfindungsgemäß besonders bevorzugt sein.

Als weiterhin vorteilhaft hat es sich gezeigt, wenn zusätzlich zu dem bzw. den Polymer(en) aus der gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- VinylpyrrolidonNinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl)-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.
Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt.

Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)ₓ-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Caicium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Schließlich können die erfindungsgemäßen Mittel auch Pflanzenextrakte (L) enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in □ - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 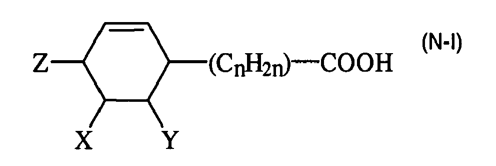 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäßebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäß verwendeten kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, - ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Die erfindungsgemäßen Mittel können auch als Haarfärbemittel, beispielsweise Oxidationshharfärbemittel oder als sogenannte Färbeshampoos formuliert werden. Färbeshampoos enthalten üblicherweise einen oder mehrere direktziehende Farbstoffe. Diese Farbstoffe können aber auch in Färbemitteln zur Nuancierung eingesetzt werden. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chior-2-nitrobenzol, 4-Amino-3-nitrophenol, 1-(2'-Ureidoethyl)amino-4-nitrobenzol, 4-Amino-2-nitrodiphenyiamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Erfindungsgemäß bevorzugte Oxidationsfärbemittel oder Färbeshampoos sind dadurch gekennzeichnet, daß sie mindestens einen direktziehenden Farbstoff enthaltender ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

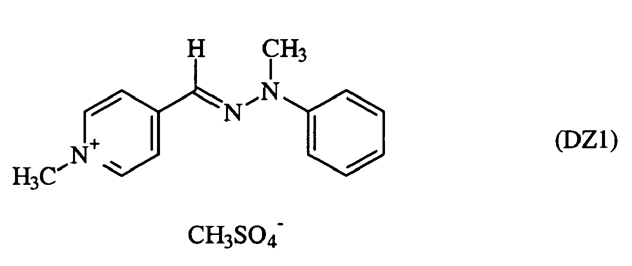

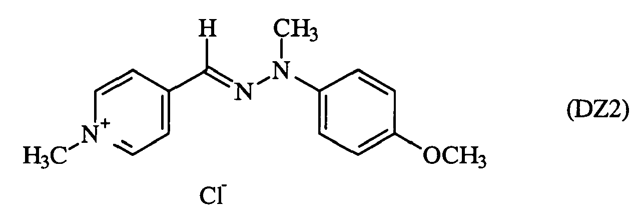

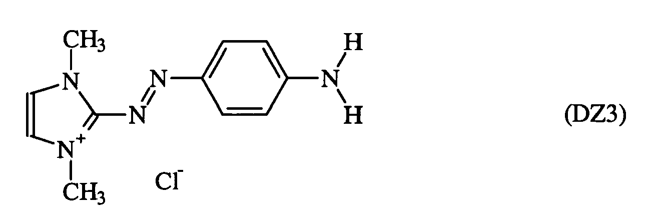

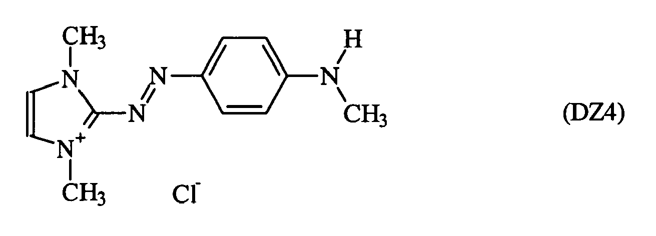

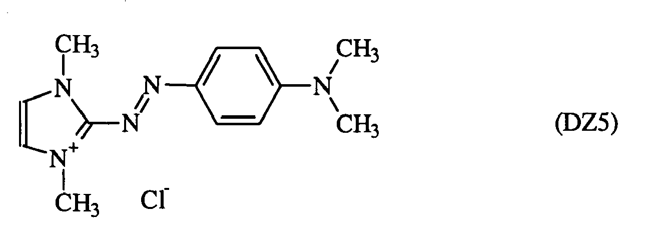

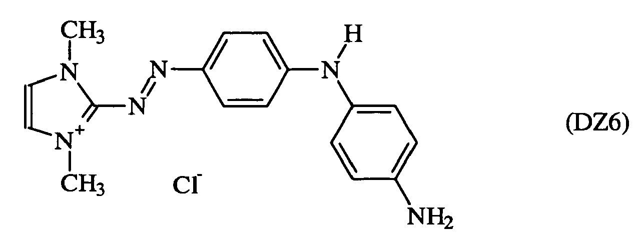

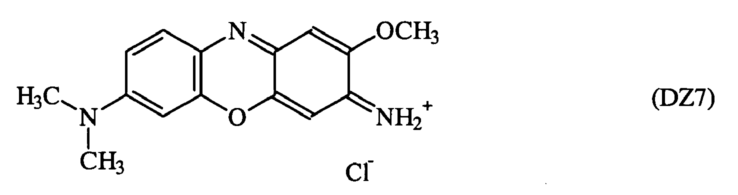

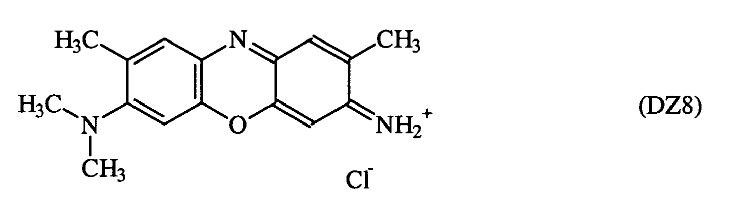

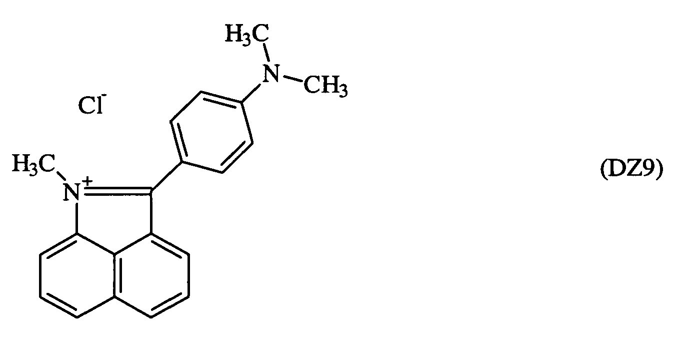

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel oder Färbeshampoo.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Mittel können als Mittel zum Färben und/oder als Mittel zum Aufhellen keratinischer Fasern konfektioniert werden. Unter keratinischen bzw. keratinhaltigen Fasern werden im Rahmen dieser Anmeldung Pelze, Wolle, Federn und insbesondere menschliche Haare verstanden.

Es ist also erfindungsgemäß möglich, ein Färbemittel (sogenanntes "Oxidationsfärbemittel") bereitzustellen, oder ein reines Blondiermittel (auch Aufhellmittel genannt). Selbstverständlich sind auch Produkte, die gleichzeitig aufhellen und färben, erfindungsgemäß herstellbar.

Erfindungsgemäße Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können erfindungsgemäße Oxidationsfärbemittel auch noch direktziehende Farbstoffe als Nuanceure enthalten.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 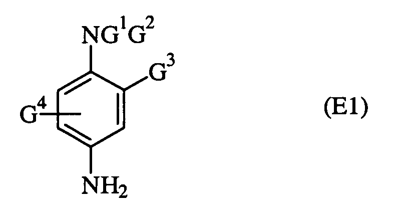 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenytendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Totuylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (E2) entsprechen, sowie ihre physiologisch verträglichen Salze: 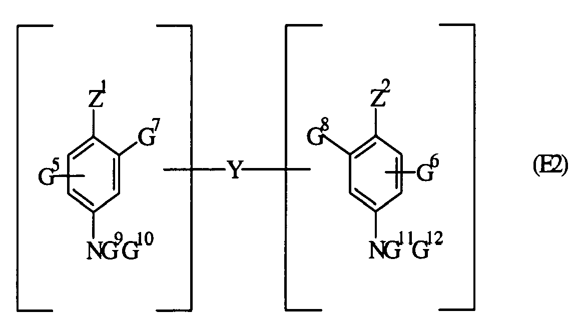 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 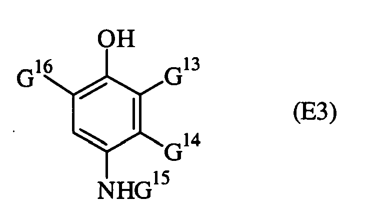 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenoi, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 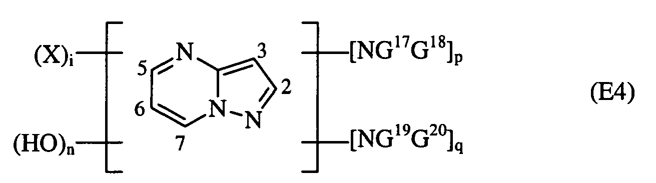 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

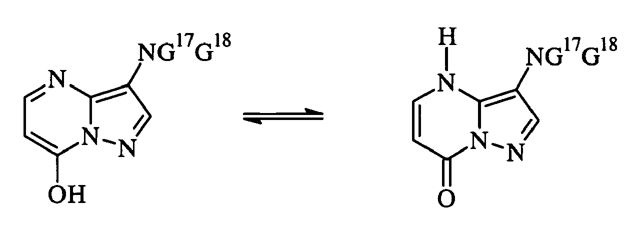

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß besonders bevorzugte Oxidationsfärbemittel sind dadurch gekennzeichnet, daß die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(ß-Hydroxyethyl)-p- phenylendiamin, N,N-Bis-(β-hydroxyethyl)-p-phenylendiamin, N,N'-bis-(ß- Hydroxyethyl)-N,N'-bis- (4'-aminophenyl)-1,3-diamino-propan-2-ol, Bis-(2-hydroxy-5- aminophenyl)- methan, N,N'-Bis-(4'-aminophenyl)-1,4-diazacycloheptan, 1,10-Bis-(2',5'- diaminophenyl)-1,4,7,10-tetraoxadecan, p-Aminophenol, 4- Amino-3-fluorphenol, 4-Amino-2-aminomethylphenol, 4- Amino-2-((diethylamino)methyl)phenol, o-Aminophenol, 2-Amino-4- methylphenol, 2-Amino-5- methylphenol, 2-Amino-4-chlorphenol, 2,4,5,6- Tetraaminopyrimidin, 4-Hydroxy-2,5,6- triaminopyrimidin, 2-Hydroxy-4,5,6- triaminopyrimidin, 2-Dimethylamino-4,5,6- triaminopyrimidin, 2,4- Dihydroxy-5,6-diaminopyrimidin, 2,5,6-Triaminopyrimidin, 1-(2'- Hydroxy- 5'-aminobenzyl)-imidazolidin-2-on, 4,5-Diamino-1-(2'-hydroxyethyl) pyrazol und *N*-(4-Amino-3-methylphenyl)-*N*-[3-(1*H*-imidazol-1-yl)propyl]amin trihydrochlorid.

Die erfindungsgemäßen Färbemittel enthalten mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methyl-resorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIIa), 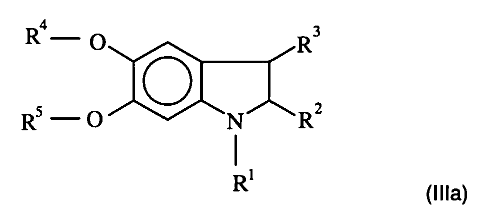 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIIb), 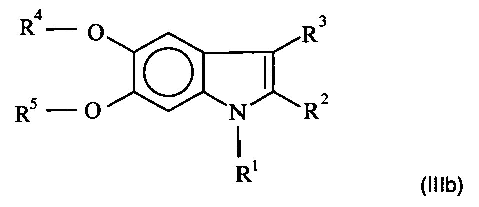 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Erfindungsgemäß besonders bevorzugte Oxidationsfärbemittel sind dadurch gekennzeichnet, daß die Kupplerkomponente ausgewählt ist aus m-Phenylendiaminderivaten, Naphtholen, Resorcin und Resorcinderivaten, Pyrazolonen, m-Aminophenolen und substituierten Pyridinderivaten, wobei bevorzugte Mittel Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin, 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 3-Amino-4-(2-methoxyethoxy)-5-methylphenylamin und/oder 2-[(3-Morpholin-4-ylphenyl)amino]ethanol dihydrochlorid enthalten.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren Ausführungsform enthält das erfindungsgemäße Mittel daher eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung
c) von Wasser unter Bildung von Hydraten als Additionsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxyacetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxybenzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-l-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrroi-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyctohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)-penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)-hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethytpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, - methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- oder Aminosubstituenten, als reaktive Carbonylverbindung verwendet. Dabei werden wiederum die Verbindungen gemäß Formel (Ca-1) bevorzugt, 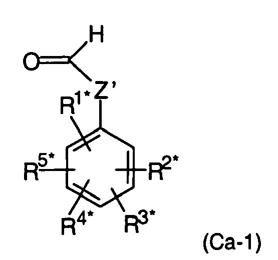 worin
- R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, eine Carbamoylgruppe, eine C₂-C₆-Acylgruppe oder eine Nitrogruppe,
- Z' steht für eine direkte Bindung oder eine Vinylengruppe,
- R^{4*} und R^{5*} stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente C werden besonders bevorzugt ausgewählt aus bestimmten Aldehyden. Hier sind erfindungsgemäße Mittel bevorzugt, die zusätzlich mindestens eine reaktive Carbonylverbindung enthalten, die ausgewählt wird, aus der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxybenzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethylbenzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxybenzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxybenzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-düod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer reaktive Carbonylverbindung(en) enthalten.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methylchinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Geeignete Verbindungen mit primärer oder sekundärerAminogruppe als Komponente B sind z.B. primäre aromatische Amine wie N,N-Dimethyl-, N,N-Diethyl-, N- (2- Hydroxyethyl)-N-ethyl-, N,N-Bis-(2-hydroxyethyl)-, N-(2-Methoxyethyl-), 2,3-, 2,4-, 2,5- Dichlor-p-phenylendiamin, 2-Chlor-p-phenylendiamin, 2,5-Dihydroxy-4- morpholinoanilin- dihydrobromid, 2-, 3-, 4-Aminophenol, 2-Aminomethyl-4-aminophenol, 2- Hydroxymethyl- 4-aminophenol, o-, p-Phenylendiamin, o-Toluyiendiamin, 2,5-Diaminotoluol, -phenot, - phenethol, 4-Amino-3-methylphenol, 2-(2,5-Diaminophenyl)-ethanoi, 2,4- Diaminophen- oxyethanoi, 2-(2,5-Diaminophenoxy)-ethanol, 4-Methylamino-, 3-Amino-4- (2'- hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2- hydroxyethylamino)-, 6-Methyl-3-amino-2-chlor-, 2-Methyl-5-amino-4-chlor-, 3, 4-Methylendi- oxy-, 5-(2-Hydroxyethylamino)-4-methoxy-2-methyl-, 4-Amino-2- hydroxymethylphenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicyisäure, 3- Amino-4-hydroxy-, 4-Amino-3-hydroxybenzoesäure, 2-, 3-, 4-Aminobenzolsulfonsäure, 3-Amino- 4- hydroxybenzolsulfonsäure, 4-Amino-3-hydroxynaphthalin-1 -sulfonsäure, 6- Amino-7- hydroxynaphthalin-2-sulfonsäure, 7-Amino-4-hydroxynaphthalin-2- sulfonsäure, 4-Amino- 5-hydroxynaphthalin-2,7-disulfonsäure, 3-Amino-2-naphthoesäure, 3- Aminophthalsäure, 5-Aminoisophthalsäure, 1,3,5-, 1,2,4-Triaminobenzol, 1,2,4,5- Tetraaminobenzol, 2,4,5- Triaminophenol, Pentaaminobenzol, Hexaaminobenzol, 2,4,6-Triaminoresorcin, 4,5- Diaminobrenzcatechin, 4,6-Diaminopyrogaliol, 3,5-Diamino-4-hydroxybrenzcatechin, aromatische Aniline bzw. Phenole mit einem weiteren aromatischen Rest, wie sie in der nachstehenden Formel dargestellt sind 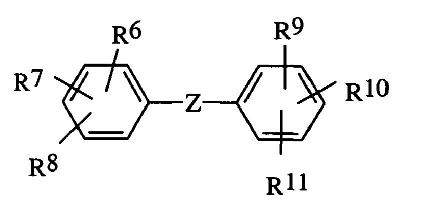 in der R6 für eine Hydroxy- oder eine Aminogruppe, die durch C1-4-Alkyl, Cl.4- Hydroxyalkyl- oderC1-4-Alkoxy-C1-4-alkyl substituiert sein kann, steht, R 7 , R", R9, R'O und Rl' für Wasserstoff, eine Hydroxy- oder eine Aminogruppe, die durch eine Cl-4-Aikyl-, Cl-4-Hydroxyalkyl, Cl-4-Aminoalkyl- oder Cl-4-Alkoxy- C1-4-alkylgruppe sub- stituiert sein kann, für eine Carbon- doer Sulfonsäuregruppe stehen, und Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel 111 Q-(CH2-p-CH2-Q')1> (111) in der P eine direkte Bindung, eine CH2- oder CHOH-Gruppe bedeutet, Q und Q' unabhängig voneinander für ein Sauerstoffatom, eine NR 12 - Gruppe, worin R 12 Wasserstoff, eine Cl-,-Alkyl-, oder Hydroxy-C 1-4-alkylgruppe bedeutet, die Gruppe 0-(CH2)p-NH oder NH-(CH2)p,-0, worin p und p' 2 oder 3 sind, stehen und o eine Zahl von 1 bis 4 bedeutet, wie beispielsweise 4,4'-Diaminostilben, 4,4'-Diaminostilben-2,2'- disulfonsäure-mono- oder -di-Na-Salz, 4-Amino-4'-dimethylaminostilben, 4,4'- Diaminodiphenylmethan, -sulfid,
-sulfoxid, -amin, 4,4'-Diaminodiphenylamin-2-sulfonsäure, 4,4'- Diaminobenzophenon, - diphenylether, 3,3',4,4'-Tetraaminodiphenyl, 3,3',4,4'-Tetraamino- benzophenon, 1,3-Bis- (2,4-diaminophenoxy)-propan, 1,8-Bis-(2,5-diaminophenoxy)-3,6-dioxaoctan, 1,3-Bis-(4-aminophenylamino)-propan, -2-propanoi, 1,3-Bis-[N-(4-aminophenyl)-2- hydroxyethyla- mino]-2-propanol, N,N-Bis-[2-(4-aminophenoxy)-ethyl]-methylamin, N- Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2- Amino-3-hydroxy-, 2,6-Diamino-, 2,5-Diamino-, 2,3-Diamino-, 2- Dimethylamino-5-amino-, 2-Methylamino-3-amino-6-methoxy-, 2,3-Diamino-6-methoxy-, 2,6-Dimethoxy- 3,5-dia- mino-, 2,4,5-Triamino-, 2,6-Dihydroxy-3,4-dimethylpyridin, 2,4-Dihydroxy- 5,6-diamino-, 4,5,6-Triamino-, 4-Hydroxy-2,5,6-triamino-, 2-Hydroxy-4,5,6-triamino-, 2, 4,5,6-Tetraamino-, 2-Methylamino-4,5,6-triamino-, 2,4-, 4,5-Diamino-, 2-Amino-4- methoxy-6-methyl- pyrimidin, 3,5-Diaminopyrazol, -1,2,4-triazol, 3-Amino-, 3-Amino-5- hydroxypyrazol, 2-,3-, 8-Aminochinolin, 4-Amino-chinaidin, 2-, 6-Aminonicotinsäure, 5-Aminoisochinolin, 5-, 6- Aminoindazol, 5-, 7-Amino-benzimidazol, -benzothiazol, 2,5-Dihydroxy-4-morpholinoani- lin sowie Indole und Indoline, sowie deren physiologisch verträgliche Salze, sein. Bevorzugte Beispiele für Indol- bzw. Indolinderivate 5,6-Dihydroxyindol, N- Methyl-5,6-dihydroxyindol, N-Ethyl-5,6-dihydroxyindol, N-Propyl-5,6-dihydroxyindol, N- Butyl-5,6-dihy- droxyindol, 5,6-Dihydroxyindol-2-carbonsäure, 6-Hydroxyindol, 6- Aminoindol und 4-Aminoindol. Weiterhin bevorzugt sind 5,6-Dihydroxyindolin, N-Methyl-5,6- dihydroxyindolin, N-Ethyl-5,6-dihydroxyindolin, N-Propyl-5,6-dihydroxyindolin, N-Butyl-5,6- dihydroxyindolin, 5,6-Dihydroxyindolin-2-carbonsäure. 6-Hydroxyindolin, 6-Aminoindolin und 4-Aminoindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen bevorzugt alle natürlich vorkommenden und synthetischen (alpha-Aminosäuren in Frage, z.B. die durch Hydrolyse aus pflanzlichen oder tierischen Proteinen, z.B.

Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Es können aber auch andere Aminosäuren verwendet werden, wie z. B. 6- Aminocapronsäure.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemittein ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5- Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2- Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4- Chlorresorcin, 1-Naphthol, 1,5-, 2,3-, 2,7-Dihydroxynaphthalin, 6- Dimethylamino-4- hydroxy-2-naphthalinsulfonsäure, 3,6-Dihydroxy-2,7-naphthalinsulfonsäure.

Als CH-aktive Verbindungen können beispielhaft genannt werden 1,2,3,3- Tetramethyl- 3H-indoliumiodid, 1,2,3,3-Tetramethyl-3H-indolium-p-toluoisulfonat, 1,2, 3,3-Tetramethyl- 3H-indoliummethansulfonat, Fischersche Base (1,3,3-Trimethyl-2- methylenindolin), 2,3- Dimethyl-benzothiazoliumiodid, 2,3-Dimethyl-benzothiazolium-p- toluolsulfonat, Rhodanin, Rhodanin-3-essigsäure, 1 -Ethyl-2-chinaldiniumiodid, 1 -Methyl-2- chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Bevorzugt werden die CH-aciden Verbindungen aus den Formeln (II) und/oder (III) und/oder (IV) ausgewählt 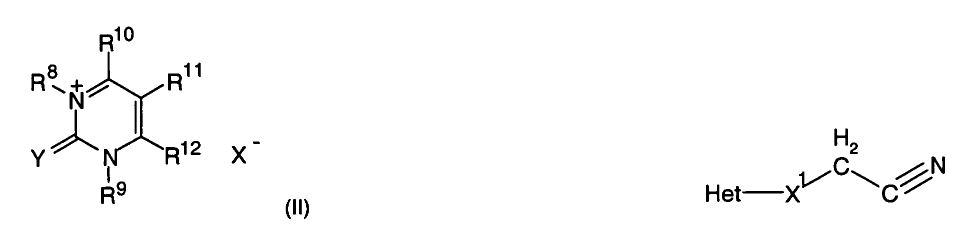 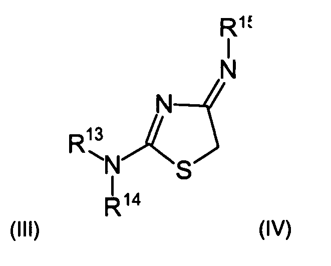 worin
- R⁸ und R⁹ stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R'R"N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine C₁-C₆-Alkylgruppe bedeutet,
- R¹¹ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
- R¹³ und R¹⁴ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R'R"N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R' und R" gemeinsam mit dem Stickstoffatom einen 5-, 6- oder 7-gliedrigen Ring bilden können und m steht für eine Zahl 2, 3, 4, 5 oder 6, und
- R¹⁵ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
- X- steht für ein physiologisch verträgliches Anion,
- Het steht für einen gegebenenfalls substituierten Heteroaromaten,
- X¹ steht für eine direkte Bindung oder eine Carbonylgruppe.

Gleichwirkend zu den Verbindungen der Formel II sind deren Enaminformen. Es wird hier ausdrücklich auf die Druckschrift WO-A1-2004/022016 verwiesen, auf die vollinhaltlich Bezug genommen wird.

Mindestens eine Gruppe R¹⁰ oder R¹² gemäß Formel II steht zwingend für eine C₁-C₆-Alkylgruppe. Diese Alkylgruppe trägt an deren alpha-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y für ein Sauerstoff- oder ein Schwefelatom, besonders bevorzugt für ein Sauerstoffatom.

Der Rest R⁸ wird bevorzugt ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

R¹¹ steht bevorzugt für ein Wasserstoffatom.

Besonders bevorzugt stehen die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

Vorzugsweise sind die Verbindungen gemäß Formel II ausgewählt aus einer oder mehrerer Verbindungen der Gruppe von Salzen mit physiologisch verträglichem Gegenion X⁻ , die gebildet wird aus Salzen des
1,2-Dihydro-1,3,4,6-tetramethyl-2-oxo-pyrimidiniums,
1,2-Dihydro-1,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

Ganz besonders bevorzugte erfindungsgemäße Mittel sind dadurch gekennzeichnet, dass sie als CH-acide Verbindung
Salze des 1,2-Dihydro-1,3,4,6-tetramethyl-2-oxopyrimidiniums,
Salze des 1,2-Dihydro-1,3,4-trimethyl-2-oxopyrimidiniums,
Salze des 1,2-Dihydro-1,3,4,6-tetramethyl-2-thioxopyrimidiniums,
Salze des 1-Allyl-1,2-dihydro-3,4,6-trimethyl-2-oxopyrimidiniums,
Salze des 1,2-Dihydro-1-(2-hydroxyethyl)-3,4,6-trimethyl-2-oxopyrimidiniums, 2-(Cyanomethyl)benzimidazol,
4,5-Dihydro-4-imino-2-(1-piperidinyl)-thiazol und/oder dessen Hydrochlorid,
4,5-Dihydro-4-imino-2-(4-morpholinyl)-thiazol und/oder dessen Hydrochlorid,
4,5-Dihydro-4-imino-2-(1-pyrrolidinyl)-thiazol und/oder dessen Hydrochlorid
enthalten.

X⁻ steht in Formel (II) sowie in obigen Listen bevorzugt für Halogenid, Benzolsulfonat, p-Toluolsulfonat, C₁-C₄-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, Iodid, Hydrogensulfat oder p-Toluolsulfonat als X- eingesetzt.

Der Rest Het gemäß Formel (III) steht bevorzugt für das Molekülfragment mit der Formel (V), 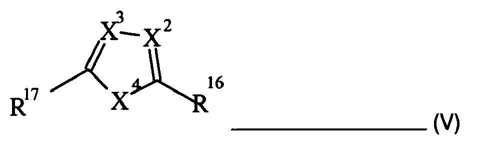 worin
- R¹⁶ und R¹⁷ stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{VIII}R^{IX}N-(CH₂)ₘ-, worin R^{VIII} und R^{IX} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{VIII} und R^{IX} gemeinsam mit dem Stickstoffatom einen 5-, 6- oder 7-gliedrigen Ring bilden können und m steht für eine Zahl 0, 1, 2, 3 oder 4,
   wobei R¹⁶ und/oder R¹⁷ einen an den Ring des Restmoleküls ankondensierten, gegebenenfalls substituierten aromatischen oder heteroaromatischen, 5- oder 6-Ring bilden können
- X² und X³ stehen unabhängig voneinander für ein Stickstoffatom oder eine Gruppe CR¹⁵, wobei R¹⁵ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{X}R^{XI}N-(CH₂)ₙ-, worin R^{X} und R^{XI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{X} und R^{XI} gemeinsam mit dem Stickstoffatom einen 5-, 6- oder 7-gliedrigen Ring bilden können und n steht für eine Zahl 0, 1, 2, 3 oder 4,
   wobei mindestens einer der Substituenten X² und X³ zusammen mit dem Restmolekül einen ankondensierten gegebenenfalls substituierten aromatischen 5- oder 6-Ring bilden kann,
- X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylguppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{XII}R^{XIII}N-(CH₂)ₚ- steht, worin R^{XII} und R^{XIII} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{XII} und R^{XIII} gemeinsam mit dem Stickstoffatom einen 5-, 6- oder 7-gliedrigen Ring bilden können und p steht für eine Zahl 0, 1, 2, 3 oder 4, substituiert sein können,
mit der Maßgabe, daß, wenn X⁴ für eine Vinylengruppe steht, mindestens eine der Gruppen X² oder X³ ein Stickstoffatom bedeutet.

Die Bindung des heterozyklischen Rings gemäß Formel (V) zum Molekülfragment -X¹-CH₂-C≡N unter Erhalt der erfindungsgemäßen Verbindung gemäß Formel (III) erfolgt an den Ring des Heterozyklusses und ersetzt ein an diesen Ring gebundenes Wasserstoffatom. Folglich ist es zwingend notwendig, daß die Substituenten R¹⁶, R¹⁷, X², X³ und X⁴ derart gewählt werden müssen, daß mindestens einer dieser Substituenten eine entsprechende Bindungsbildung ermöglicht. Folglich ist es zwingend, daß mindestens einer der Reste R¹⁶ oder R¹⁷ die Bindung zum Molekülfragment -X¹-CH₂-C=N ausbildet, wenn X⁴ ein Sauerstoffatom oder ein Schwefelatom ist und X² und X³ ein Stickstoffatom bedeuten.

Der Rest Het gemäß Formel (III) wird besonders bevorzugt abgeleitet von den Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Benzothiazol, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hydroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise sind die Verbindungen gemäß Formel (III) ausgewählt aus der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrroi-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril. Besonders bevorzugt ist 1*H*-Benzimidazol-2-ylacetonitril [2-(Cyanomethyl)benzimidazol].

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer CH-acider Verbindung(en) enthalten.

Die erfindungsgemäßen Mittel weisen vorteilhafte Eigenschaften auf und verleihen den mit ihnen behandelten Körperpartien ebenfalls vorteilhafte Eigenschaften. Insbesondere bei der Haar- und Kopfhautbehandlung wurden Vorteile beobachtet. So steigern erfindungsgemäße Haarbehandlungsmittel die Elastizität und den Glanz der mit ihnen behandelten Haare und führen zu einer mechanischen Festigung Haarfasern, welche sich z.B. in einer Verbesserung der Naß- und Trockenkämmbarkeiten sowie eine Verhinderung frühzeitiger Splißbildung bei den behandelten Haaren zeigt.

Auf der Haut und insbesondere der Kopfhaut bewirken die erfindungsgemäßen Mittel eine Erhöhung der Elastizität und überraschenderweise sebumregulierende Effekte. Der optische Eindruck "fettiger" Haut oder Haare wird damit vermieden bzw. abgeschwächt.

Ein weiterer Gegenstand der vorliegenden Erfindung ist die Verwendung von mindestens einem Silikon der Formel Si-I

**(CH₃)₃Si-[O-Si(CH₃)₂]ₓ-O-Si(CH₃)₃** **(Si-I),**

in der x für eine Zahl von 0 bis 5000, vorzugsweise von 10 bis 2500, weiter
bevorzugt von 50 bis 1500 und insbesondere 100 bis 1000, steht, und
in Kombination mit mindestens einem Silikon der Formel Si-II

**(CH₃)₃Si-[O-Si(CH₃)₂]ₓ-[O-(H₃C)Si((CH₂)ₖ(CH₂CH₂O)ₘ(CH₂CH₂CH₂O)ₙ)]_{y}-O-Si(CH₃)₃** **(Si-II),**

in der
- k für Werte von 1 bis 20, vorzugsweise von 2 bis 10 und insbesondere für 2, 3, 4, 5, 6 steht;
- m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
- n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
- x bzw. y jeweils unabhängig voneinander für eine Zahl von 0 bis 5000, vorzugsweise von 10 bis 2500, weiter bevorzugt von 50 bis 1500 und insbesondere 100 bis 1000 stehen,
mit der Maßgabe, daß (m + n) nicht Null ist,
in kosmetischen Mitteln, insbesondere in Haarbehandlungsmitteln.

Bevorzugte erfindungsgemäße Verwendungen dienen der
- Steigerung der Elastizität keratinischer Fasern, insbesondere menschlicher Haare und/oder,
- Verbesserung der Glätte keratinischer Fasern, insbesondere menschlicher Haare und/oder,
- Verbesserung des Glanzes keratinischer Fasern, insbesondere menschlicher Haare und/oder,
- Verbesserung der Naß- und/oder Trockenkämmbarkeit keratinischer Fasern, insbesondere menschlicher Haare und/oder,
- Verbesserung des Schutzes keratinischer Fasern, insbesondere menschlicher Haare vor mechanischer Belastung und/oder,
- Verbesserung der Abscheidung von Silikonen der Formel Si-I auf keratinischen Fasern, insbesondere menschlichen Haaren und/oder,
- Verbesserung der Abscheidung von Silikonen der Formel Si-II auf keratinischen Fasern, insbesondere menschlichen Haaren.

Bezüglich weiterer bevorzugter Ausführungsformen der erfindungsgemäßen Verwendung gilt mutatis mutandis das zu den erfindungsgemäßen Mitteln Gesagte.

## Patentansprüche

1. Kosmetisches Mittel, **dadurch gekennzeichnet, daß** es - bezogen auf sein Gewicht -
a) 0,01 bis 10 Gew.-% mindestens eines Silikons der Formel Si-I
**(CH₃)₃Si-[O-Si(CH₃)₂]ₓ-O-Si(CH₃)₃** **(Si-I),**
in der x für eine Zahl von 0 bis 5000, vorzugsweise von 10 bis 2500, weiter bevorzugt von 50 bis 1500 und insbesondere 100 bis 1000, steht, und
b) 0,01 bis 10 Gew.-% mindestens eines Silikons der Formel Si-II
**(CH₃)₃Si-[O-Si(CH₃)₂]ₓ-[O-(H₃C)Si((CH₂)ₖ(CH₂CH₂O)ₘ(CH₂CH₂CH₂O)ₙ)]_{y}-O-Si(CH₃)₃** **(Si-II),**
in der
- k für Werte von 1 bis 20, vorzugsweise von 2 bis 10 und insbesondere für 2, 3, 4, 5, 6 steht;
- m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
- n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
- x bzw. y jeweils unabhängig voneinander für eine Zahl von 0 bis 5000, vorzugsweise von 10 bis 2500, weiter bevorzugt von 50 bis 1500 und insbesondere 100 bis 1000 stehen"
mit der Maßgabe, daß (m + n) nicht Null ist
enthält.

2. Kosmetisches Mittel nach Anspruch 1, **dadurch gekennzeichnet, daß** es - bezogen auf sein Gewicht - 0,02 bis 8,5 Gew.-%, vorzugsweise 0,1 bis 7,5 Gew.-%, besonders bevorzugt 0,25 bis 5 Gew.-%, weiter bevorzugt 0,25 bis 4 Gew.-% und insbesondere 0,3 bis 2,5 Gew.-% mindestens eines Silikons der Formel Si-I enthält.

3. Kosmetisches Mittel nach einem der Ansprüche 1 oder 2, **dadurch gekennzeichnet, daß** es - bezogen auf sein Gewicht - 0,02 bis 8,5 Gew.-%, vorzugsweise 0,1 bis 7,5 Gew.-%, besonders bevorzugt 0,25 bis 5 Gew.-%, weiter bevorzugt 0,25 bis 4 Gew.-% und insbesondere 0,3 bis 2,5 Gew.-% mindestens eines Silikons der Formel Si-II enthält.

4. Kosmetisches Mittel nach einem der Ansprüche 1 bis 3, **dadurch gekennzeichnet, daß** es als Silikon der Formel Si-I ein Dimethicone enthält, das eine Viskosität von 10 bis 1.000.000 cSt, vorzugsweise von 100 bis 600.000 cSt, besonders bevorzugt von 1000 bis 300.000 cSt, weiter bevorzugt von 5000 bis 200.000 cSt und insbesondere von 10.000 bis 70.000 cSt (gemessen bei 20°C) aufweist.

5. Kosmetisches Mittel nach einem der Ansprüche 1 bis 4, **dadurch gekennzeichnet, daß** es als Silikon der Formel Si-II ein Dimethicone Copolyol enthält, das eine Viskosität von 1 bis 10.000 cSt, vorzugsweise von 10 bis 5000 cSt, besonders bevorzugt von 50 bis 2500 cSt, weiter bevorzugt von 100 bis 1000 cSt und insbesondere von 300 bis 500 cSt (gemessen bei 20°C) aufweist.

6. Kosmetisches Mittel nach einem der Ansprüche 1 bis 5, **dadurch gekennzeichnet, daß** mindestens 25 Gew.-%, vorzugsweise mindestens 50 Gew.-%, besonders bevorzugt mindestens 75 Gew.-%, weiter bevorzugt mindestens 95 Gew.-% und insbesondere 100 Gew.-% der im Mittel enthaltenen Silikone der Formel Si-I ausgewählt ist/sind aus Silikonen der Formel Si-I
(CH₃)₃Si-[O-Si(CH₃)₂]ₓ-O-Si(CH₃)₃ (Si-I)
in der x für eine Zahl aus der Gruppe 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, 900, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1018, 1019, 1020, 1021, 1022, 1023, 1024, 1025, 1026, 1027, 1028, 1029, 1030, 1031, 1032, 1033, 1034, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1058, 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1083, 1084, 1085, 1086, 1087, 1088, 1089, 1090, 1091, 1092, 1093, 1094, 1095, 1096, 1097, 1098, 1099, 1100 steht.

7. Kosmetisches Mittel nach einem der Ansprüche 1 bis 6, **dadurch gekennzeichnet, daß** das Gewichtsverhältnis der im Mittel enthaltenen Silikone der Formel Si-I zu den im Mittel enthaltenen Silikonen der Formel Si-II 10:1 bis 1:10, vorzugsweise 8:1 bis 1:8, besonders bevorzugt 7:1 bis 1:6, weiter bevorzugt 4:1 bis 1:3 und insbesondere 3:1 bis 1:2 beträgt.

8. Kosmetisches Mittel nach einem der Ansprüche 1 bis 7, **dadurch gekennzeichnet, daß** es - bezogen auf sein Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthält, wobei bevorzugte kationische(s) Polymer(e) ausgewählt ist/sind aus
p. Poly(methacryloyloxyethyltrimethylammoniumchlorid) (INCl: Polyquaternium-37) und/oder;
q. quaternisierten Cellulose-Derivaten (INCl: Polyquaternium 10) und/oder
r. kationischen Alkylpolyglycosiden und/oder
s. kationisertem Honig und/oder
t. kationischen Guar-Derivaten und/oder
u. polymeren Dimethyldiallylammoniumsalzen und deren Copolymeren mit Estern und Amiden von Acrylsäure und Methacrylsäure und/oder
v. Copolymeren des Vinylpyrrolidons mit quaternierten Derivaten des Dialkylaminoalkylacrylats und -methacrylats und/oder
w. Vinylpyrrolidon-Vinylimidazoliummethochlorid-Copolymeren und/oder
x. quaterniertem Polyvinylalkohol und/oder
y. Polyquaternium 2 und/oder
z. Polyquaternium-7 und/oder
aa. Polyquaternium 17 und/oder
bb. Polyquaternium 18 und/oder
cc. Polyquaternium 24 und/oder
dd. Polyquaternium 27.

9. Kosmetisches Mittel nach einem der Ansprüche 1 bis 8, **dadurch gekennzeichnet, daß** es zusätzlich Tensid(e), vorzugsweise anionische(s) Tensid(e), besonders bevorzugt Fettalkoholethersulfate der Formel
**H₃C-(CH₂)ₙ-(OCH₂CH₂)ₖ-OSO₃⁻ M⁺**
in der n für Werte von 5 bis 21, vorzugsweise von 7 bis 19, besonders bevorzugt von 9 bis 17 und insbesondere von 11 bis 13 und k für Werte von 1, 2, 3, 4 oder 4, vorzugsweise für 2, und M für ein Kation aus der Gruppe Na⁺, K⁺ NH₄⁺, ½ Mg²⁺, ½ Zn²⁺, vorzugsweise für Na⁺, stehen, wobei bevorzugte Mittel das bzw. die Tensid(e) in Mengen von 2 bis 20 Gew.-%, vorzugsweise von 5 bis 15 Gew.-% und insbesondere von 7,5 bis 12 Gew.-%, jeweils bezogen auf das gesamte Mittel, enthalten.

10. Kosmetisches Mittel nach einem der Ansprüche 1 bis 9, **dadurch gekennzeichnet, daß** es zusätzlich amphotere(s) Tensid(e), vorzugsweise aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betaine bekannten Verbindungen,
- der unter der INCI-Bezeichnung Coco-Betaine bekannten Verbindungen,
- der unter der INCI-Bezeichnung Disodium Cocoamphodiacetate bekannten Verbindungen,
wobei bevorzugte Mittel das bzw. die amphotere(n) Tensid(e) in Mengen von 1 bis 15 Gew.-%, vorzugsweise von 2,5 bis 12 Gew.-% und insbesondere von 5 bis 10 Gew.-%, jeweils bezogen auf das gesamte Mittel, enthalten.

11. Kosmetisches Mittel nach einem der Ansprüche 1 bis 8, **dadurch gekennzeichnet, daß** es zusätzlich- bezogen auf sein Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin und/oder Purinderivat(e) der Formel (I) enthält 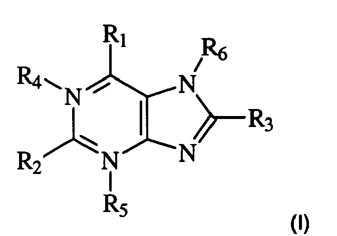 in der die Reste R¹, R² und R³ unabhängig voneinander ausgewählt sind aus -H, - OH, - NH₂, -SH und die Reste R⁴, R⁵ und R⁶ unabhängig voneinander ausgewählt sind aus -H, - CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶= H)
- 6-Thioguanin (R¹ = SH, R² = NH₂, R³ R⁴ = R⁵ = R⁶ = H)
- Xanthin (R¹ = R² = OH, R³ = R⁴ = R⁵ R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = H)
- Theophyllin (R¹ = R² = OH, R³ = H, R⁴ = CH₃, R⁵ = CH₃, R⁶ = H).

12. Kosmetisches Mittel nach einem der Ansprüche 1 bis 11, **dadurch gekennzeichnet, daß** es zusätzlich - bezogen auf sein Gewicht - 0,05 bis 4,5 Gew.-%, vorzugsweise 0,1 bis 4 Gew.-%, besonders bevorzugt 0,5 bis 3,5 Gew.-% und insbesondere 0,75 bis 2,5 Gew.-% Kohlenhydrat(e) enthält, wobei bevorzugte Kohlenhydrate ausgewählt sind aus
- Monosachhariden, insbesondere
- D-Ribose und/oder
- D-Xylose und/oder
- L-Arabinose und/oder
- D-Glucose und/oder
- D-Mannose und/oder
- D-Galactose und/oder
- D-Fructose und/oder
- Sorbose und/oder
- L-Fucose und/oder
- L-Rhamnose
- Disacchariden, insbesondere
- Saccharose und/oder
- Maltose und/oder
- Lactose und/oder
- Trehalose und/oder
- Cellobiose und/oder
- Gentiobiose und/oder
- Isomaltose.

13. Kosmetisches Mittel nach einem der Ansprüche 1 bis 12, **dadurch gekennzeichnet, daß** es zusätzlich - bezogen auf sein Gewicht - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthält.

14. Kosmetisches Mittel nach einem der Ansprüche 1 bis 13, **dadurch gekennzeichnet, daß** es zusätzlich Vitamine und/oder Pro-Vitamine und/oder Vitaminvorstufen enthält, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

15. Kosmetisches Mittel nach einem der Ansprüche 1 bis 14, **dadurch gekennzeichnet, daß** es zusätzlich 0,05 bis 15 Gew.-% mindestens eines 2-Furanonderivats der Formel (I) und/oder der Formel (II) 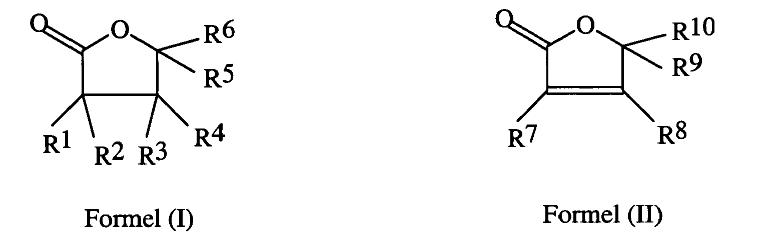 enthält, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂- C₄- gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

16. Kosmetisches Mittel nach einem der Ansprüche 1 bis 15, **dadurch gekennzeichnet, daß** es zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 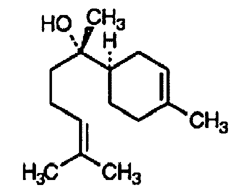 enthält.

17. Verwendung von mindestens einem Silikon der Formel Si-I
**(CH₃)₃Si-[O-Si(CH₃)₂]ₓ-O-Si(CH₃)₃** **(Si-I),**
in der x für eine Zahl von 0 bis 5000, vorzugsweise von 10 bis 2500, weiter bevorzugt von 50 bis 1500 und insbesondere 100 bis 1000, steht, und
in Kombination mit mindestens einem Silikon der Formel Si-II
**(CH₃)₃Si-[O-Si(CH₃)₂]ₓ-[O-(H₃C)Si((CH₂)ₖ(CH₂CH₂O)ₘ(CH₂CH₂CH₂O)ₙ)]_{y}-O-Si(CH₃)₃** **(SI-II),**
in der
- k für Werte von 1 bis 20, vorzugsweise von 2 bis 10 und insbesondere für 2, 3, 4, 5, 6 steht;
- m für Werte von 0 bis 100, vorzugsweise von 2 bis 50, besonders bevorzugt von 5 bis 35 und insbesondere für 8, 9, 10, 11, 12, 13, 14, 15, 16, 17, 18, 19, 20 steht;
- n für Werte von 0 bis 100, vorzugsweise von 0 bis 50, besonders bevorzugt von 0 bis 35 und insbesondere für 0, 8, 9, 10, 11, 12, 13, 14, 15, 16, 17, 18, 19, 20 steht;
- x für eine Zahl von 0 bis 5000, vorzugsweise von 10 bis 2500, weiter bevorzugt von 50 bis 1500 und insbesondere 100 bis 1000 steht,
mit der Maßgabe, daß (m + n) nicht Null ist,
in kosmetischen Mitteln, insbesondere in Haarbehandlungsmitteln.

18. Verwendung nach Anspruch 17 zur
- Steigerung der Elastitizität keratinischer Fasern, insbesondere menschlicher Haare und/oder,
- Verbesserung der Glätte keratinischer Fasern, insbesondere menschlicher Haare und/oder,
- Verbesserung des Glanzes keratinischer Fasern, insbesondere menschlicher Haare und/oder,
- Verbesserung der Naß- und/oder Trockenkämmbarkeit keratinischer Fasern, insbesondere menschlicher Haare und/oder,
- Verbesserung des Schutzes keratinischer Fasern, insbesondere menschlicher Haare vor mechanischer Belastung und/oder,
- Verbesserung der Abscheidung von Silikonen der Formel Si-I auf keratinischen Fasern, insbesondere menschlichen Haaren und/oder,
- Verbesserung der Abscheidung von Silikonen der Formel Si-II auf keratinischen Fasern, insbesondere menschlichen Haaren.
